(19) Europäisches Patentamt
European Patent Office
Office européen des brevets

(11) EP 0 799 188 B1

(12) EUROPEAN PATENT SPECIFICATION

(45) Date of publication and mention of the grant of the patent:
**15.09.1999 Bulletin 1999/37**

(21) Application number: **95942141.3**

(22) Date of filing: **13.12.1995**

(51) Int Cl.[6]: **C07C 233/47**, C07C 323/59, C07H 13/06, C07D 207/16, A61K 31/195, A61K 31/22, A61K 31/70, A61K 31/40

(86) International application number:
**PCT/EP95/04926**

(87) International publication number:
**WO 96/18600 (20.06.1996 Gazette 1996/28)**

# (54) AMIDES OF MONO AND DICARBOXYLIC ACIDS WITH GLYCOSAMINES, SELECTIVELY ACTIVE ON THE CANNABINOID PERIPHERAL RECEPTOR

AMIDE VON MONO-UND DICARBONSÄUREN MIT GLYCOSAMINEN, MIT SELEKTIVER WIRKUNG AUF DEN PERIPHEREN CANNABINOIDEN REZEPTOR

AMIDES D'ACIDES MONO ET BICARBOXYLIQUES A GLYCOSAMINES, A ACTIVITE SELECTIVE SUR LE RECEPTEUR PERIPHERIQUE DES CANNABINOIDES

(84) Designated Contracting States:
**AT BE CH DE DK ES FR GB GR IE IT LI LU MC NL PT SE**

(30) Priority: **14.12.1994 IT MI942513**

(43) Date of publication of application:
**08.10.1997 Bulletin 1997/41**

(73) Proprietor: **LIFEGROUP S.p.A.**
**I-35043 Monselice (Padova) (IT)**

(72) Inventors:
- **DELLA VALLE, Francesco**
  **I-35122 Padova (IT)**
- **LEON, Alberta**
  **I-35142 Padova (IT)**
- **MARCOLONGO, Gabriele**
  **I-35020 Carrara S. Giorgio (IT)**
- **LORENZI, Silvana**
  **I-35142 Padova (IT)**

(74) Representative: **Gervasi, Gemma, Dr.**
**Studio Brevetti e Marchi**
**NOTARBARTOLO & GERVASI S.r.l.,**
**Corso di Porta Vittoria, 9**
**20122 Milano (IT)**

(56) References cited:
**EP-A- 0 552 405**
**WO-A-93/21913**
**WO-A-94/12466**
**US-A- 4 933 363**
**US-A- 4 999 417**

- **CHEMICAL ABSTRACTS, vol. 89, no. 14, 2 October 1978 Columbus, Ohio, US; abstract no. 117553, MORI, MOTOO ET AL: "Stabilized hair dye compositions" XP002001421 & JP,A,53 059 041 (KANEBO, LTD., JAPAN)**
- **J. MED. CHEM. (1977), 20(11), 1362-71 , XP002001420 ACTON, EDWARD M. ET AL: "Antitumor septacidin analogs"**
- **CHEMICAL ABSTRACTS, vol. 118, no. 20, 17 May 1993 Columbus, Ohio, US; abstract no. 194576, ZHU, JIANGUANG ET AL: "4RO-X collectors for flotation of calciferous minerals" XP002001422 & FEIJINSHUKUANG (1991), (4), 19-22 ,**
- **CHEMICAL ABSTRACTS, vol. 75, no. 9, 30 August 1971 Columbus, Ohio, US; abstract no. 61382, RUEDE, E. ET AL: "Immunological properties of synthetic sugar-polypeptide conjugates. Effect of N-lauroylglucosamine residues on immunogenicity" XP002001423 & EUR. J. IMMUNOL. (1971), 1(2), 113-23 ,**
- **CHEMICAL ABSTRACTS, vol. 70, no. 9, 3 March 1969 Columbus, Ohio, US; abstract no. 38057, SCIORTINO, T. ET AL: "Antiviral activity. VIII. Higher N-acyl derivatives of some amino acids" XP002001424 & BOLL. CHIM. FARM. (1968), 107(8), 498-505,**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

(Cont. next page)

- CHEMICAL ABSTRACTS, vol. 100, no. 8, 20 February 1984 Columbus, Ohio, US; abstract no. 55086, SWIATKOWSKI, P. ET AL: "Enrichment of alkaline earth metal-containing minerals from their gangues" XP002001425 & SE,A,429 822 (KENOGARD AB, SWED.)
- CHEMICAL ABSTRACTS, vol. 121, no. 26, 26 December 1994 Columbus, Ohio, US; abstract no. 307976, KITAHARA, TAKASHI ET AL: "hair preparations or skin cosmetics containing carboxylic acids, their salts esters or amido derivatives as sebum secretion inhibitors" XP002001426 & JP,A,06 192 032 (KAO CORP, JAPAN)
- CHEMICAL ABSTRACTS, vol. 111, no. 13, 25 September 1989 Columbus, Ohio, US; abstract no. 111948, RINALDO, PIERO ET AL: "Stable isotope dilution analysis of n-hexanoylglycine, 3-phenylpropionylglycine and suberylglycine in human urine using chemical ionization gas chromatography/mass spectrometry selected ion monitoring" XP002001427 & BIOMED. ENVIRON. MASS SPECTROM. (1989), 18(7), 471-7,
- CHEMICAL ABSTRACTS, vol. 104, no. 13, 31 March 1986 Columbus, Ohio, US; abstract no. 103706, GILLOIS, M. ET AL: "Lipid A monosaccharide analogs inhibiting oxidative phosphorylation" XP002001428 & ANN. INST. PASTEUR/MICROBIOL. (1985), 136B(2), 125-34 ,
- CHEMICAL ABSTRACTS, vol. 89, no. 13, 25 September 1978 Columbus, Ohio, US; abstract no. 110297, GREGERSEN, NIELS ET AL: "Gas chromatographic mass spectrometric identification of N-dicarboxylmonoglycines" XP002001429 & BIOMED. MASS SPECTROM. (1978), 5(1), 80-3 CODEN: BMSYAL;ISSN: 0306-042X, 1978,
- CHEMICAL ABSTRACTS, vol. 82, no. 23, 9 June 1975 Columbus, Ohio, US; abstract no. 156723, KONDO, KIYOSHI ET AL: "N-(.beta.-Hydroxyalkanoyl).omega.-amino acids and esters" XP002001430 & JP,A,49 127 917 (SAGAMI CHEMICAL RESEARCH CENTER)
- CHEMICAL ABSTRACTS, vol. 82, no. 23, 9 June 1975 Columbus, Ohio, US; abstract no. 156724, KONDO, KIYOSHI ET AL: "N-(.beta.-Hydroxyalkanoyl).alpha.-amino acids and esters" XP002001431 & JP,A,49 127 918 (SAGAMI CHEMICAL RESEARCH CENTER)
- CHEMICAL ABSTRACTS, vol. 117, no. 17, 26 October 1992 Columbus, Ohio, US; abstract no. 172062, GRIGORYAN, N. A. ET AL: "Synthesis and antistaphylococcal activity of dicarboxylic acid derivatives containing an amino acid fragment" XP002001432 & KHIM.-FARM. ZH. (1992), 26(2), 43-5 ,
- MOL. PHARMACOL. (1995), 48(2), 288-92, XP000569156 PRILLER, JOSEF ET AL: "Mead ethanolamide, a novel eicosanoid, is an agonist for the central (CB1) and peripheral (CB2) cannabinoid receptors"
- PROSTAGLANDINS, LEUKOTRIENES ESSENT. FATTY ACIDS (1995), 53(4), 301-8, XP000569157 FONTANA, A. ET AL: "Analysis of anandamide, an endogenous cannabinoid substance, and of other natural N-acylethanolamines"

## Description

The present invention refers to new amides of mono- and dicarboxylic acids with aminoalcohols, therapeutically active in the treatment of pathologies connected with the modulation of the peripheral receptor for cannabinoids.

## STATE OF THE ART

Cannabinoids are a specific class of psycho-active compounds present in hemp (Cannabis sativa), comprising more than 60 different molecules, the most important of which are: cannabinol, cannabidiol and many isomers of tetrahydrocannabinol. The therapeutic activity of Cannabis is known since the old Chinese dynasties which used it already 5000 years ago in the treatment of asthma, migraine and some gynaecological disorders. The use was confirmed along the years and in 1850 the Cannabis extracts where entered the American Pharmacopoeia and maintained up to 1947.

Cannabinoids are responsible of many effect on different systems and/or organs; the most relevant effects are on the central nervous system and on the cardiovascular system. In fact, they affect mood, memory, motory coordination and cognition, increase heart rate and modify the systemic arterious pressure. It is also known that cannabinoids are capable of lowering the intraocular pressure and produce effects on the respiratory and endocrine system (L.E. Hollister, Health Aspects of Cannabis, Pharmacological Reviews, 38, 1-20. 1986). More recently also their capacity of suppressing the cellular and humoral immune response and their antiinflammatory properties were described (A. W. Wirth et al., Antiinflammatory properties of Cannabichromene, Life Science 26, 1991-95, 1980). However, the use of Cannabis in therapy is debated, because of its relevant psychoactive effects, which gives addiction and dependence, and its various side effects which up to now are not completely known (L.E. Hollister, 1986, cited above).

In spite of the large use made along the centuries, the mechanism of action of cannabinoids was for long unknown. Only in 1990 Matsuda et al. could identify and clone a receptor for cannabinoids which was discovered to be a member of the super-family of the G-protein-coupled receptors; CB1 is coupled to G1 to inhibit adenilate cyclase activity and to a pertussis-sensitive G protein to regulate $Ca^{2+}$ currents. Such receptor is mainly localised in the brain, in cell-lines of nervous origin and only to a limited extent at peripheral level; therefore, depending on its location, it was named Central Receptor (CB1) (Matsuda et al., Structure of a cannabinoid receptor and functional expression of the cloned cDNA, Nature 346:561-564, 1990). The discovery of a receptor led to the assumption that the existence of a specific endogenous ligand. Other studies permitted to isolate in the pig brain a subtonic capable of producing an antagonist effect, i.e. capable of competitively binding to the cannabinoid central receptor. The structural study and the comparative tests with the synthetic product permitted to identify such substance, which resulted to be an amidic derivative of arachidonic acid, and more particularly the arachidonylethanolamide, later named anandamide. The pharmacological characterization of such molecule showed that anandamide possesses an activity profile similar, although less potent, than Δ9-THC (tetrahydrocannabinol with a double bond in position 9), and is able to mimic its psychoactive effects. Such results brought to the conclusion that anandamide is the endogenous ligand of the central receptor for cannabinoids (C. C. Felder et al. Anandamide, an endogenous cannabimimetic eicosanoid, binds to the cloned human cannabinoid receptor and stimulates receptor-mediated signal transduction, PNAS, 90, 7656-7660,1993; P.B. Smith et al. The pharmacological Activity of Anandamide, a Putative Endogenous Cannabinoid, in Mice, J. PET. 270, 219-227, 1994).

Following studies permitted to identify substances capable of binding to the receptor CB1, which were grouped in a class of amidic compounds named anandamides by the authors (Hanus L. et al. Two New Unsatured Fatty Acid Ethanolamides in Brain That Bind to the Cannabinoid Receptor, J. Med. Chem., 36, 3032-3034, 1993). The discovery that the ethanolamide of arachidonic acid, but not the ethanolamide of other acids which are biologically important and anyway endogenously present at the cerebral level (such as palmitic acid), is capable of functionally activating CB1 central receptor, brought about the subsequent identification of other amides of ethanolamine with highly unsaturated fatty acids which have an affinity to CB1 receptor. Its peculiar distribution led to assume the existence of differentiated receptor sites. In fact, a second different receptor for cannabinoids was cloned, named Peripheral Receptors (CX5 or CB2). Since such receptor was identified in the spleen and macrophages/monocytes, being on the other hand absent at the central level, it is considered that this receptor mediates the non psycho-active effects of the cannabinoids (S. Munro et al., Molecular characterisation of a peripheral receptor for cannabinoids, Nature, 365, 61-65, 1993). In this connection there are some evidences of the capacity of Δ9-THC to induce immunosuppressive effects. Recent experimental studies demonstrated that Δ9-THC is capable of influencing the function of macrophage. Exposition to Δ9-THC lowers the cytolytic action of activated macrophages, measured as synthesis, release and citotoxicity of TNF-α. Anyhow, since the macrophages release various molecules having a cytolytic potential, other than TNF-α, it is considered that they can represent a target for Δ9-THC (K. Fischer-Stenger et al.,Δ9-tetrahydrocannabinol Inhibition of Tumor necrosis Factor-alpha: Suppression of Post-translational Events, J. PET, 267, 1558-1565, 1993). All these evidences and the preferential massive localisation of CB2 receptor in the immuno system confirm that such receptor plays a

specific role in mediating the immune and antiinflammatory response to stimuli of different nature, included the bacterial and the viral ones.

It was also demonstrated that anandamide, endogenous ligand for the CB1 central receptor, is capable of binding to the CB2 receptor with an affinity which is 30 folds inferior to that of the central receptor; this probably implies the existence of a different endogenous ligand for such receptor, up to now still unknown (L. L. Iversen, Medical uses of marijuana?, Nature, 365, 12-13, 1993). As already mentioned, the therapeutical use of cannabinoids as analgesics, antiemetics, anticonvulsants, antispastics, antiglaucoma and, more recently, antiinflammatory, is limited by the presence of undesired side effects, or psychoactive effects, and by the outcome of addiction and pharmacological tolerance (W.L. Dewey, Cannabinoid Pharmacology, Pharmacological Reviews, 38, 151-178, 1986). Recently some compounds were prepared, capable of acting as agonists on both the cannabinoids receptors: for example it is known the use of derivatives of dihydropyrrole-(1,2,3-d,e)-1,4-benzoxazine in the treatment of glaucoma (US 5 112 820, Derwent abstract) and it is also known the use of derivatives of 1,5-diphenyl-pyrazole as immunomodulators or psychotropic agents in the treatment of various neuropathologies, migraine, epilepsy, glaucoma ecc. (European Patent Application EP 576 357, Derwent abstract).

However, such compounds, being active on both CB1 central and CB2 peripheral receptor, can lead to the outcome of serious psychoactive effects, a part from addiction and dependence.

In the light of the fact that the cannabinoids act through a receptorial mechanism and especially on receptors capable of mediating different functional effects, and also in view of the low homology between the Peripheral and Central Receptors, it is evident the importance of developing a class of drugs selectively active on the receptor subtype and not indiscriminately on both the receptors such as the natural or synthetic cannabinoids.

The search on the pharmacological effects mediated by cannabinoids receptors shows that the non-psychoactive effects of Cannabis derivatives are mediated by CB2 peripheral receptor. Furthermore, receptor CB2 localisation confirms that such non-psychoactive effects, i.e. the effects on the immune system and the antiinflammatory, myorelaxant and antinociceptive effects, as well as the influence on the pressure systems, are mediated by such receptor.

It is therefore extremely important to obtain compounds which are capable of selectively acting on the peripheral receptor for cannabinoids and, therefore, on the pathologies connected with the modulation of such receptor, excluding the psychoactive effects at central level and the relevant side effects related to such action, such as addiction and dependence.

## SUMMARY OF THE INVENTION

The Applicant has now found a class of amidic derivatives of aliphatic mono and dicarboxylic acids with aminoalcohols, able to bind selectively to the cannabinoid peripheral receptor CB2 and to activate functionally the same; said amidic derivatives have formula (I):

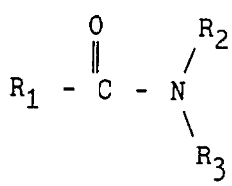

(I)

wherein $R_1$ is:

1) a linear or branched hydrocarbon radical having from 9 to 23 carbon atoms, preferably from 11 to 17, saturated or presenting one double bond, optionally substituted with one or more -OH groups;
2) a group of formula (II):

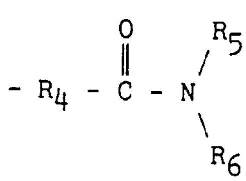

(II)

wherein $R_4$ is a linear hydrocarbon radical, saturated or containing one double bond, comprising from 7 to 22

carbon atoms, preferably from 10 to 16, optionally substituted with one or more linear or branched alkyl groups $C_1$-$C_8$ and/or with one or more -OH groups;

3) a group of formula (IV):

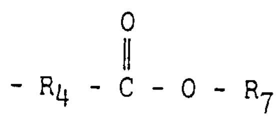 (IV)

wherein $R_4$ has the meanings described above and $R_7$ is -H or a linear or branched alkyl group comprising from 1 to 20 carbon atoms.

In formula (I) the residue of formula (III):

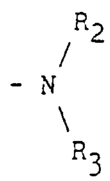 (III)

represents a glycosaminic residue of formula:

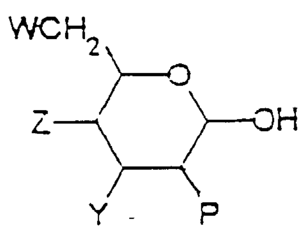

wherein one of the groups P, Y and Z is

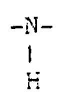 or 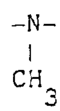 ,

and the remaining groups, including W, are -H or -OH.

In formula (II) the residue of formula (V):

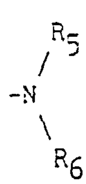 (V)

can be the same as (III), defined above, with $R_5$ and $R_6$ equal or different from $R_2$ and $R_3$.

The residues of formulae (III) and (V), can be optionally homogeneously substituted on $R_2$, $R_3$, $R_5$ and/or $R_6$ with at least one pharmaceutically acceptable radical capable of increasing the solubility of the compounds of the present invention in water, alcohols and suitable organic solvents. Said pharmaceutically acceptable radical are selected from the group consisting of acyls (i.e. acetyl and benzoyl), hemiacyls of dicarboxylic acids (i.e. succinic and glutaric acid), amino-acyles, dialkylamino-acyls, alkyl-sulphonates (i.e. metansulphonate and p-toluen-sulphonate),

alkyls (i.e. methyl and ethyl), O-phosphates and O-sulphates.

Therefore, it is an object of the present invention the use of the described derivatives as active principles in the preparation of pharmaceutical compositions for the treatment of pathologies of mammals connected with the modulation of the cannabinoid peripheral receptor.

A further object of the present invention are new amides of formula (I), wherein $R_1$ has the meanings decribed above and the residues of formulae (III) and (V) are residues of a glycosamine, as reported with the proviso that said amide of formula (I) is different from 2-deoxy-2-[(3-hydroxy-1-oxotetradecyl)-amino]-α-D-glucopyranose.

Other objects of the present invention are the process for the preparation of the above described new amides and the pharmaceutical compositions containing therapeutically active quantities of said new amides and of their more soluble and/or slow release form, for the therapeutic treatment of pathologies connected with the modulation of the cannabinoid peripheral receptor.

## DESCRIPTION OF THE DRAWINGS

Figure 1 illustrates the specific hybridization of cannabinoid peripheral receptor amplified by Polymerase Chain Reaction (PCR) in A) rat spleen, B) rat peritoneal mast cell cultures and C) RBL-2H3 cell cultures.

Figure 2 illustrates the specific mRNA-CB2 cannabinoid peripheral receptor detected by in situ hybridisation in Cerebellar granule cells (Fig. 2A) and Cerebellum (Fig, 2B).

## DETAILED DESCRIPTION OF THE INVENTION

The advantages and characteristics of the pharmaceutical use of the class of amidic derivatives of mono or dicarboxylic acids with aminoalcohols, selectively active on the cannabinoid peripheral receptor, the new amides, the process for their preparation and the pharmaceutical compositions containing them, according to the present invention, will be better illustrated in view of the following detailed description.

The amides of mono and dicarboxylic acids with aminoalcohols according to the present invention have formula (I):

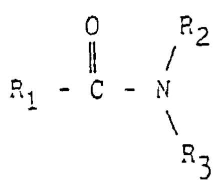

(I)

When $R_1$ belongs to class (1), it forms, with the adjacent carbonyl, the acyl group of a monocarboxylic acid, preferably lauric, myristic, palmitic, stearic, palmitoleic, oleic acid and their homologues substituted with hydroxyl groups, as for example ω-hydroxypalmitic acid.

When $R_1$ belongs to classes (2) and (3), $R_4$ forms, together with the two adjacent carbonyls, the acyl group of a dicarboxylic acid, preferably traumatic acid.

Are known in the state of the art the compounds of formula (I) wherein $R_1$ belongs to class (1) and $R_2$ forms, together with the nitrogen atom to which it is bound, the aminoacylic residue of alanine, β-alanine and glycine, and $R_3$ = $CH_3$ (International Application WO 93/21913). Furthermore, are known in the state of the art many compounds of formula (I), wherein $R_1$ has the meanings described above and the residues of formulae (III) and (V) belong to class (A)(European Patent Application EP 0 552 405; Chemical Abstract, 1993, (Volume 118,n.194576r). On the opposite, to the Applicant's knowledge, are new in the art the amides of formula (I), wherein $R_1$ has the meanings described above and the residues of formulae (III) and (V) are the residues of a glycosamine, as reported.

Nevertheless, it is known in the state of the art the compound 2-deoxy-2-[(3-hydroxy-1-oxotetradecyl)-amino]-α-D-glucopyranose with (Chemical Abstract, 1986, Volume 104, n. 103706e). In formula (III) the glycosamine is preferably D-Glucosamine, L-Acosamine, D-Mannosamine, D-Galactosamine.

The process for the preparation of the new amides of formula (I), according to the invention, involves the reaction of an activated form of the acid $R_1$-COOH, of formula $R_1$-CO-X, with an amine of formula

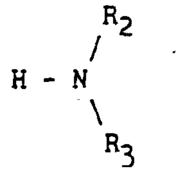

in an aqueous basic medium or in organic solvents in the presence of bases, under stirring at a temperature ranging from -20° to 60°C, and preferably from -10° to 20°C, for a period ranging from 10 minutes to 24 hours, and preferably from 1 to 3 hours.

After the optional acidification of the reaction medium, the desired product is isolated and purified by conventional techniques. Said activated form of the acid can be an acyl halide, a succinimideester, an acylisourea, a p-$NO_2$-phenylester or a methylester; said base is preferably $K_2CO_3$, KOH, $Et_3N$ or N-methyl-morpholine.

The following examples of preparation of the amides of mono and dicarboxylic acids according to the present invention are reported for illustrative but not limitative purposes.

**EXAMPLE 1: Preparation of N-palmitoyl-L-serine**

1.58 g of L-serine (15 mmol) are solubilized at 4°C in 60 ml of $K_2CO_3$ 1M. The solution is slowly added, dropwise under stirring, over a period of 30 minutes, with 2.75 g of palmitoyl chloride (10 mmol). The resulting mixture is maintained under stirring at 0°C overnight, thereafter is acidified with hydrochloric acid 6N. The precipitated crude product is separated by filtration, dried under vacuum and crystallized a first time from 30 ml of ter-butyl-methyl-ether and thereafter from 30 ml of cold methanol. The crystallized product is separated by filtration, washed twice with 5 ml methanol and dried under high vacuum.

The reaction yield is about 89%.

The physico-chemical properties of N-palmitoyl-L-serine are as follows:

- physical state: white crystalline powder
- molecular formula: $C_{19}H_{37}NO_4$
- molecular weight: 343.51
- elemental analysis: C= 66.43%. H= 10.86% N= 4.08% O= 18.63%
- solubility in org. sol.: >10 mg/ml in DMSO
  >10 mg/ml in ethanol
- water solubility: slightly soluble
- melting point: 97-99°C
- TLC: eluent: chloroform/methanol/water/
  $NH_3$ (28%) 80:25:2:1 Rf = 0.15

**EXAMPLE 2: Preparation of N-lauroyl-L-serine**

1.58 g of L-serine (15 mmol) are solubilized at 4°C in 40 ml of KOH 1N. The solution is slowly added, dropwise under stirring over a period of 30 minutes, with 2.19 g of lauroyl chloride (10 mmol). The resulting mixture is maintained under stirring at 0°C overnight, thereafter is acidified with hydrochloric acid 6N and extracted three times with 20 ml ethyle acetate; the organic phases are collected, dried with anhydrous sodium sulphate and evaporated under vacuum. The crude product is crystallized from 30 ml of cold acetonitrile. The crystallized product is separated by filtration, washed twice with 5 ml acetonitrile and dried under high vacuum.

The reaction yield is about 85%.

The physico-chemical properties of N-lauroyl-L-serine are as follows:

- physical state: white crystalline powder
- molecular formula: $C_{15}H_{29}NO_4$
- molecular weight: 287.40
- elemental analysis: C= 62.69% H= 10.17% N= 4.87% O= 22.27%
- solubility in org. sol.: >10 mg/ml in DMSO
  >10 mg/ml in ethanol
- water solubility: slightly soluble (> 10 mg/ml as sodium salt)
- melting point: 120-122°C

- TLC: eluent: toluene/ethanol/acetic acid 65:30:5 Rf = 0.46

**EXAMPLE 3: Preparation of N-oleoyl-L-serine**

1.58 g of L-serine (15 mmol) are solubilized at 4°C in 60 ml of $K_2CO_3$ 1M. The solution is slowly added, dropwise under stirring over a period of 30 minutes, with 3.01 g of oleoyl chloride (10 mmol). The resulting mixture is maintained under stirring at 0°C overnight, thereafter is acidified with hydrochloric acid 6N. The precipitated crude product is separated by filtration, dried under vacuum and purified by silica-gel column chromatography using as eluent a mixture of chloroform/methanol/water/acetic acid 90:10:0.5:0.1. The fractions of eluate containing the product are collected, evaporated to dryness and the residue is dried under high vacuum.

The reaction yield is about 88%.

The physico-chemical properties of N-oleoyl-L-serine are as follows:

- physical state: white amorphous powder
- molecular formula: $C_{21}H_{39}NO_4$
- molecular weight: 369.54
- elemental analysis: C= 68.26% H= 10.64% N= 3.79% O= 17.32%
- solubility in org. sol.: >10 mg/ml in DMSO
- water solubility: slightly soluble
- melting point: -
- TLC: eluent: chloroform/methanol/water/ $NH_3$ (28%) 80:25:2:1 Rf = 0.14

**EXAMPLE 4: Preparation of N-palmitoleyl-L-serine**

1.58 g of L-serine (15 mmol) are solubilized at 4°C in 60 ml of $K_2CO_3$ 1M. The solution is slowly added, dropwise under stirring over a period of 30 minutes, with 2.73 g of palmitoyl chloride (10 mmol). The resulting mixture is maintained under stirring at 0°C overnight, thereafter is acidified with hydrochloric acid 6N and extracted three times with 20 ml ethyle acetate; the organic phases are washed twice with 15 ml of water, collected and evaporated under vacuum. The crude product is purified by preparative silica-gel column chromatography, using as eluent a mixture of chloroform/methanol/water/acetic acid 90:10:0.5:0.1. The eluate fractions containing the product are collected, evaporated to dryness and the residue is dried under high vacuum.

The reaction yield is about 87%.

The physico-chemical properties of N-palmitoleyl-L-serine are as follows:

- physical state: white amorphous powder
- molecular formula: $C_{19}H_{35}NO_4$
- molecular weight: 341.49
- elemental analysis: C= 66.83% H= 10.33% N= 4.10% O= 18.74%
- solubility in org. sol.: >10 mg/ml in DMSO
- water solubility: slightly soluble
- melting point: -
- TLC: eluent: toluene/ethanol/acetic acid 65:30:5 Rf: 0.45

**EXAMPLE 5: Preparation of N-lauroyl-L-serine methylester**

2.33 g of L-serine methylester hydrochloride (15 mmol) are solubilized at 4°C in 40 ml of a mixture tetrahydrofurane/water 9:1 with 4.05 g of triethylamine (40 mmol). The solution is slowly added, dropwise under stirring over a period of 30 minutes, with 2.19 g of lauroyl chloride (10 mmol). The resulting mixture is maintained under stirring at 0°C for 2 h and at room temperature for a night and evaporated to dryness under vacuum. The crude product is suspended in 50 ml of hydrochloric acid 1N, extracted three times with 20 ml ethyle acetate; the organic phases are washed twice with 15 ml water, collected, dried on anhydrous sodium sulphate and evaporated under vacuum. The crude product is crystallized from 30 ml of cold hexane. The crystallized product is separated by filtration, washed twice with 5 ml hexane and dried under high vacuum.

The reaction yield is about 87%.

The physico-chemical properties of N-lauroyl-L-serine methylester are as follows:

- physical state: white crystalline powder
- molecular formula: $C_{16}H_{31}NO_4$
- molecular weight: 301.43
- elemental analysis: C= 63.75% H= 10.37% N= 4.65% O= 21.23%
- solubility in org. sol.: >10 mg/ml in DMSO >10 mg/ml in ethanol
- water solubility: slightly soluble
- melting point: 63-65°C
- TLC: eluent: toluene/ethanol/acetic acid 65:30:5 Rf= 0.60

**EXAMPLE 6: Preparation of N-palmitoyl-L-cysteine**

1.82g of L-cysteine (15 mmol) are solubilized at 4°C in 60 ml of $K_2CO_3$ 1M. The solution is slowly added, dropwise under stirring over a period of 30 minutes, with 2.75 g of palmitoyl chloride (10 mmol). The resulting mixture is maintained under stirring at 0°C overnight, thereafter is acidified with hydrochloric acid 6N. The crude precipitate is separated by filtration, dried under vacuum and crystallized a first time from 30 ml of ter-butyl-methyl-ether and thereafter from 30 ml of cold methanol. The crystallized product is separated by filtration, washed twice with 5 ml methanol and dried under high vacuum.
The reaction yield is about 92%.
The physico-chemical properties of N-palmitoyl-L-cysteine are as follows:

- physical state: white crystalline powder
- molecular formula: $C_{19}H_{37}NO_3S$
- molecular weight: 359.57
- elemental analysis: C= 63.47%, H= 10.37%, N= 3.90%, O= 13.35%, S= 8.92%
- solubility in org. sol.: >10 mg/ml in DMSO
- water solubility: slightly soluble
- melting point: -
- TLC: eluent: chloroform/methanol/water/ $NH_3$ (28%) 80:25:2:1 Rf: 0.42

**EXAMPLE 7: Preparation of N-palmitoyl-glycine**

1.13 g of glycine (15 mmol) are solubilized at 4°C in 40 ml of KOH 1N. The solution is slowly added, dropwise under stirring over a period of 30 minutes, with 2.75 g of palmitoyl chloride (10 mmol). The resulting mixture is maintained under stirring at 0°C overnight, thereafter is acidified with hydrochloric acid 6N. The crude precipitate is separated by filtration, dried under vacuum and crystallized from 60 ml of cold ethanol 95°. The crystallized product is separated by filtration, washed twice with 5 ml ethanol 95° and dried under high vacuum.
The reaction yield is about 79%.
The physico-chemical properties of N-palmitoyl-glycine are as follows:

- physical state: white crystalline powder
- molecular formula: $C_{18}H_{35}NO_3$
- molecular weight: 313.48
- elemental analysis: C= 68.97% H= 11.25% N= 4.47% O= 15.31%
- solubility in org. sol.: >10 mg/ml in DMSO
- water solubility: slightly soluble
- melting point: 121-123°C
- TLC: eluent: chloroform/methanol/water $NH_3$ (28%) 80:25:2:1 Rf: 0.22

**EXAMPLE 8: Preparation of N-lauroyl-glycine**

1.13 g of glycine (15 mmol) are solubilized at 4°C in 40 ml of KOH 1N. The solution is slowly added, dropwise under stirring over a period of 30 minutes, with 2.19 g of lauroyl-chloride (10 mmol). The resulting mixture is maintained under stirring at 0°C overnight, thereafter is acidified with hydrochloric acid 6N and extracted three times with 20 ml ethyl acetate; the organic phases are collected, dried with anhydrous sodium sulphate and evaporated under vacuum. The crude product is crystallized from 30 ml of cold acetonitrile. The crystallized product is separated by filtration, washed twice with 5 ml acetonitrile and dried under high vacuum.
The reaction yield is about 83%.
The physico-chemical properties of N-lauroyl-glycine are as follows:

- physical state: white crystalline powder
- molecular formula: $C_{14}H_{27}NO_3$
- molecular weight: 257.37
- elemental analysis: C= 65.34% H= 10.57% N= 5.44% O= 18.65%
- solubility in org. sol.: >10 mg/ml in DMSO >10 mg/ml in ethanol
- water solubility: slightly soluble (>10 mg/ml in phosphate buffer 50 mM, pH 7.4, NaCl 0.9%)
- melting point: 117-119°C
- TLC: eluent: toluene/ethanol/acetic acid 65:30:5 Rf = 0.56

**EXAMPLE 9: Preparation of N-lauroyl-glycine methylester**

1.88 g of glycine methyl ester hydrochloride (15 mmol) are solubilized at 4°C in 40 ml of a mixture tetrahydrofurane/water 9:1 with 4.05 g of triethylamine (40 mmol). The solution is slowly added, dropwise under stirring over a period of 30 minutes, with 2.19 g of lauroyl chloride (10 mmol). The resulting mixture is maintained under stirring at 0°C for 2 h and at room temperature for a night and evaporated to dryness under vacuum. The crude product is suspended in 50 ml of hydrochloric acid 1N, extracted three times with 20 ml ethyle acetate; the organic phases are washed twice with 15 ml water, collected, dried on anhydrous sodium sulphate and evaporated under vacuum. The crude is crystallized from 30 ml of cold hexane. The crystallized product is separated by filtration, washed twice with 5 ml hexane and dried under high vacuum.
The reaction yield is about 89%.
The physico-chemical properties of N-lauroyl-glycine methylester are as follows:

- physical state: white crystalline powder
- molecular formula: $C_{15}H_{29}NO_3$
- molecular weight: 271.40
- elemental analysis: C= 66.38% H= 10.77% N= 5.16% O= 17.69%
- solubility in org. sol.: >10 mg/ml in DMSO >10 mg/ml in ethanol
- water solubility: slightly soluble
- melting point: 62-64°C
- TLC: eluent: toluene/ethanol/acetic acid 65:30:5 Rf= 0.63

**EXAMPLE 10: Preparation of N-palmitoyl-β-alanine**

1.34g of β-alanine (15 mmol) are solubilized at 4°C in 40 ml of KOH 1N. The solution is slowly added, dropwise under stirring over a period of 30 minutes, with 2.75 g of palmitoyl chloride (10 mmol). The resulting mixture is maintained under stirring at 0°C overnight, acidified with hydrochloric acid 6N and extracted three times with 20 ml ethyle acetate; the organic phases are washed twice with 15 ml water, collected, dried on anhydrous sodium sulphate and evaporated under vacuum. The crude product is crystallized from 30 ml of ter-butyl-methyl-ether. The crystallized product is separated by filtration, washed twice with 10 ml ter-butyl-methyl-ether and dried under high vacuum.
The reaction yield is about 93%.
The physico-chemical properties of N-palmitoyl-β-alanine are as follows:

- physical state: white crystalline powder
- molecular formula: $C_{19}H_{37}NO_3$
- molecular weight: 327.51
- elemental analysis: C= 69.68% H= 11.39% N= 4.28% O= 14.66%
- solubility in org. sol.: >5 mg/ml in DMSO; >5 mg/ml in ethanol
- water solubility: slightly soluble
- melting point: 120 - 122°C
- TLC: eluent: toluene/ethanol/acetic acid 65:30:5 Rf: 0.64

**EXAMPLE 11: Preparation of N-palmitoyl-gamma-aminobutyric acid**

1.55 g of gamma-amino-butyric acid (15 mmol) are solubilized at 4°C in 40 ml of KOH 1N. The solution is slowly added, dropwise under stirring over a period of 30 minutes, with 2.75 g of palmitoylchloride (10 mmol). The resulting mixture is maintained under stirring at 0°C overnight, acidified with hydrochloric acid 6N and extracted three times with 20 ml ethyle acetate; the organic phases are washed twice with 15 ml water, collected, dried with anhydrous sodium sulphate and evaporated under vacuum. The crude product is crystallized from 30 ml of ter-butyl-methyl-ether.

The crystallized product is separated by filtration, washed twice with 10 ml ter-butyl-methyl-ether and dried under high vacuum.

The reaction yield is about 90%.

The physico-chemical properties of N-palmitoyl-gamma-aminobutyric acid are as follows:

- physical state: white crystalline powder
- molecular formula: $C_{20}H_{39}NO_3$
- molecular weight: 341.54
- elemental analysis: C= 70.34% H= 11.51% N= 4.10% O= 14.05%
- solubility in org. sol.: >5 mg/ml in DMSO; >5 mg/ml in ethanol
- water solubility: slightly soluble
- melting point: 104 - 106°C
- TLC: eluent: toluene/ethanol/acetic acid 65:30:5 Rf: 0.64

**EXAMPLE 12: Preparation of N,N' -bis- (1-carboxy-2-hydroxyethyl)-nonandiamide**

4.66 g of L-serine methylester hydrochloride (30 mmol) are solubilized at 4°C in 80 isopropanole with 8.1 g of triethylamine (80 mmol). The solution is slowly added, dropwise under stirring over a period of 30 minutes, with 2.25 g of azelaoyl chloride (10 mmol). The resulting mixture is maintained under stirring at 0°C for 2 h and at room temperature for a night and evaporated to dryness under vacuum. The crude product is suspended in 30 ml of water, extracted three times with 20 ml ethyle acetate; the organic phases are washed twice with 15 ml water, collected, and evaporated under vacuum. The crude product is suspended in 50 ml of water, under continuous stirring, and added with 20 ml NaOH 1M to hydrolize the esteric groups. After 30 minutes the solution is cooled to 4°C and acidified with hydrochloric acid 6N. The precipitated crude is separated by filtration, crystallized from 30 ml of a cold mixture acetonitrile/water 9: 1. The crystallized product is separated by filtration, washed twice with 5 ml acetonitrile and dried under high vacuum.

The reaction yield is about 75%.

The physico-chemical properties of N-N'-bis-(1-carboxy-2-hydroxyethyl)-nonandiamide are as follows:

- physical state: white crystalline powder
- molecular formula: $C_{15}H_{26}N_2O_8$
- molecular weight: 362.38
- elemental analysis: C= 49.72% H= 7.23% N= 7.73% O= 35.32
- solubility in org. sol.: >10 mg/ml in DMSO >10 mg/ml in ethanol
- water solubility: slightly soluble (>10 mg/ml as sodium salt)
- melting point: 89-93°C
- TLC: eluent: acetonitrile/water/acetic acid 4:1:0.1 Rf= 0.31

**EXAMPLE 13: Preparation of N,N'-trans-2-dodecendioyl-diglycine**

2.28 g of traumatic acid (10 mmol) are solubilized in 40 ml of anhydrous dimethylformamide at 0°C with 2 ml of pyridine. 2.53 g of N-hydroxysuccinimide (22 mmol) and 4.12 g of dicyclohexylcarbodiimide (20 mmol) are added and the resulting mixture is stirred for 2 h at 0°C. The formed urea is separated by filtration and discarded, while the solution containing the succinimidic ester is stirred again at 0°C and added with 2.25 g of glycine (30 mmol) and 3.04 g of triethylamine (30 mmol).

The resulting mixture is stirred for a night at room temperature and evaporated to dryness under vacuum. The residue is solubilized in 50 ml of water and the solution is acidified with hydrochloric acid 6N. The crude precipitate is collected by filtration, dried under vacuum and crystallized from 60 ml of methanol. The crystallized product is separated by filtration, washed twice with 5 ml of methanol and dried under high vacuum.

The reaction yield is 80%.

The physico-chemical properties of N,N'-trans-2-dodecendioyl-diglycine are as follows:

- physical state: white crystalline powder
- molecular formula: $C_{16}H_{26}N_2O_6$
- molecular weight: 342.39
- elemental analysis: C= 56.13% H= 7.65% N= 8.18% O= 28.04
- solubility in org. sol.: >10 mg/ml in DMSO
- water solubility: slightly soluble (>10 mg/ml as sodium salt)
- melting point: 194°C (dec)

- TLC: eluent: acetonitrile/water/formic acid 4:1:0.1 Rf= 0.85

**EXAMPLE 14: Preparation of N-palmitoyl-D-glucosamine**

3.23 g of D-glucosamine hydrochloride (15 mmol) are solubilized at 4°C in 60 ml of $K_2CO_3$ 1M. The solution is slowly added, dropwise under stirring over a period of 30 minutes, with 2.75 g of palmitoyl chloride (10 mmol). The resulting mixture is maintained under stirring at 0°C overnight and acidified to neutrality with hydrochloric acid 6N. The precipitated crude product is separated by filtration and dried under vacuum; thereafter it is purified with repeated washing with warm ethanol, recovered by filtration and dried under high vacuum.

The reaction yield is about 78%.

The physico-chemical properties of N-palmitoyl-D-glucosamine are as follows:

- physical state: white amorphous powder
- molecular formula: $C_{22}H_{43}NO_6$
- molecular weight: 417.58
- elemental analysis: C= 63.28% H= 10.38% N= 3.35% O= 22.99%
- solubility in org. sol.: >1 mg/ml in DMSO
- water solubility: slightly soluble
- melting point: 202°C (dec)
- TLC: eluent: chloroform/methanol/water $NH_3$ (28%) 80:25:2:1 Rf: 0.30

**EXAMPLE 15: Preparation of N-lauroyl-D-glucosamine**

3.23g of D-glucosamine hydrochloride (15 mmol) are solubilized at 4°C in 60 ml of $K_2CO_3$ 1M. The solution is slowly added, dropwise under stirring over a period of 30 minutes, with 2.19 g of lauroyl chloride (10 mmol). The resulting mixture is maintained under stirring at 0°C overnight and acidified to neutrality with hydrochloric acid 6N. The precipitated crude product is separated by filtration and dried under vacuum; thereafter it is purified with repeated washing with warm isopropanol, recovered by filtration and dried under high vacuum. The reaction yield is about 85%.

The physico-chemical properties of N-lauroyl-D-glucosamine are as follows:

- physical state: white amorphous powder
- molecular formula: $C_{18}H_{35}NO_6$
- molecular weight: 361.48
- elemental analysis: C= 59.81% H= 9.76% N= 3.87% O= 26.56%
- solubility in org. sol.: >5 mg/ml in DMSO
- water solubility: slightly soluble
- melting point: >170°C (dec)
- TLC: eluent: toluene/ethanol/acetic acid 65:30:5 Rf: 0.31

**EXAMPLE 16: Preparation of N-oleoyl-D-glucosamine**

3.23 g of D-glucosamine hydrochloride (15 mmol) are solubilized at 4°C in 30 ml of $K_2CO_3$ 1M. The solution is slowly added, dropwise under stirring over a period of 30 minutes, with 3.01 g of oleoyl chloride (10 mmol). The resulting mixture is maintained under stirring at 0°C overnight, acidified with hydrochloric acid 6M and extracted in continuous with ethyle acetate. The extraction solution is evaporated

to dryness and the crude product is purified by silica gel chromatography, using as eluent a mixture of chloroform/methanol/water/$NH_3$ (25%) 85:15:1:0.5; the fractions containing the pure product are collected, evaporated to dryness and the residue is dried under high vacuum.

The reaction yield is about 86%.

The physico-chemical properties of N-oleoyl-D-glucoseamine are as follows:

- physical state: white amorphous powder
- molecular formula: $C_{24}H_{45}NO_6$
- molecular weight: 443.62
- elemental analysis: C= 64.98% H= 10.22% N= 3.16% O= 21.64%
- solubility in org. sol.: >5 mg/ml in DMSO
- water solubility: slightly soluble
- melting point: -

- TLC: eluent: chloroform/methanol/water $NH_3$ (25%) 80:25:2:1 Rf: 0.35

**EXAMPLE 17: Preparation of N-lauroyl-4-hydroxy-L-proline**

1.97 g of 4-hydroxy-L-proline (15 mmol) are solubilized at 4°C in 50 ml of water containing 3.18 g of $Na_2CO_3$ (30 mmol). The solution is slowly added, dropwise under stirring over a period of 30 minutes, with 2.19 g of lauroylchloride (10 mmol) in 50 ml of petroleum ether. The resulting mixture is kept under stirring for hours at 0°C and overnight at room temperature. The resulting mixture is acidified with HCl 6N and extracted three times with 50 ml ethyle acetate; the organic phases are washed 2 times with 15 ml of water, collected, dried with anhydrous sodium sulphate and evaporated under vacuum. The crude product is crystallized from 50 ml of cyclohexane. The crystallized product is separated by filtration, washed twice with 10 ml cyclohexane and dried under vacuum.

The reaction yield is about 85%.

The physico-chemical properties of N-lauroyl-4-hydroxy-L-proline are as follows:

- physical state: white crystalline powder
- molecular formula: $C_{17}H_{31}NO_4$
- molecular weight: 313.44
- elemental analysis: C= 65.14% H= 9.97% N= 4.47% O= 20.42%
- solubility in org. sol.: >10 mg/ml in DMSO >10 mg/ml in ethanol
- water solubility: slightly soluble (>10 mg/ml in phosphate buffer 100 mM, pH 7.4)
- melting point: 86.5-88.5°C
- TLC: eluent: chloroform/methanol/water/$NH_3$ (28%) 80:25:2:1 Rf = 0.22

**EXAMPLE 18: Preparation of N-palmitoyl-D-glucosamine tetra-Oemisuccinate**

4.18 g of N-palmitoyl-D-glucosamine (10 mmol) are solubilized at 4°C in 40 ml of anhydrous pyridine; the resulting solution is added with 4.4 g of succinic anhydride (40 mmol) and kept under stirring at 0°C over 2 hours and overnight at 40°C. The solution is evaporated to dryness under vacuum, the residue is taken up with 20 ml HCl 1N and extracted 3 times with 50 ml of ethyle acetate; the organic phases are washed 2 times with 15 ml of water, collected, dried with anhydrous sodium sulphate and concentrated under vacuum to a volume of about 50 ml. The resulting solution is added with petroleum ether to muddiness and cooled at 4°C. The obtained crystallized product is separated by filtration, washed 2 times with 10 ml of petroleum ether and dried in high vacuum.

The reaction yield is about 93%.

The physico-chemical properties of N-palmitoyl-D-glucosamine tetra-O-emisuccinate are as follows:

- physical state: white crystalline powder
- molecular formula: $C_{38}H_{59}NO_{18}$
- molecular weight: 817.88
- elemental analysis: C= 55.81% H= 7.27% N= 1.71% O= 35.21%
- solubility in org. sol.: >10 mg/ml in DMSO >10 mg/ml in ethanol
- water solubility: slightly soluble (>10 mg/ml in phosphate buffer 100 mM. pH 7.4)
- melting point: 131°C (dec.)
- TLC: eluent: toluene/ethanol/acetic acid 80:20:2 Rf = 0.27

As already mentioned, the amides of mono and dicarboxylic acids with aminoalcohols and aminoethers according to the present invention, corresponding to formula (I) reported hereinabove, can selectively bind to CB2 peripheral receptor, acting as competitive agonists and displacing from said receptor the known natural and synthetic cannabinoids already known. Furthermore, said amides are capable of functionally activating CB2 receptor, by mimicking the non-psychoactive biological effects of cannabinoids and acting with a potency higher than or comparable with the one of cannabinoids.

The Applicant has also surprisingly found that CB2 cannabinoid peripheral receptor is present on mast cell and therefore the new compounds according to the present invention act on the same mast cell functionality. Furthermore, the Applicant has unexpectedly found that CB2 cannabinoid peripheral receptor is functionally expressed also on non-immune cells or tissue; in particular the Applicant has found that CB2 is functionally expressed in the Nervous System on cerebellar granule cells, indicating that CB2 receptor can modulate also non-immune cell function.

This extremely important aspect of the present invention will be illustrated in detail hereinbelow.

**A) Identification of CB2 receptor on mast cells and non-immune cells.**

In order to verify that CB2 cannabinoid peripheral receptor is present on mast cells and non-immune cells (i. e. neurons), the presence of messenger RNA for said receptor was investigated in primary cultures of rat peritoneal mast cells, in a basophilic-mastocyte cell line from rat solid tumour (Rat Basophilic Leukemia, RBL-2H3) and in mouse cerebellar granule cell culture, in comparison with rat spleen homogenate tissues, where the presence of said receptor is acknowledged.
Both Polymerase Chain Reaction and in situ hybridisation technique were used.

**i) Cannabinoid Peripheral Receptor specific hybridisation amplified by Polimerase Chain Reaction (PCR).**

Extraction of total mRNA from tissues and cells

Total RNA was extracted from rat peritoneal mast cells, RBL-2H3 cells and rat spleen, according to the method described by Chumczynski and Sacchi (Anal. Biochem., 162, 156-159, 1987).
$5 \cdot 10^6$ mast cells, cells RBL-2H3 and 50 mg of spleen were separately homogenized in a 1.5 ml polypropylene tube Eppendorf® (safe-lock type) with guanidinium isothiocyanate 5M (600 µl). Each tube was then supplied with sodium acetate 2M (60 µl), pH 5, and phenol (600 µl) saturated in buffer Tris- HCl 1M, pH 7.0. The samples were briefly stirred by vortex mixing (Heidolpn, type REAX 2000®) and then added with chloroform-isoamyl alcohol (49:1 v/v) (120 µl). After agitation for 15 sec. by vortex mixing and incubation in ice for 15 min, the samples were centrifuged at 15,000xg at 4°C for 15 min. 600 µl of the upper aqueous phase was withdrawn and placed in another tube, having care to avoid any contact with the interface of the organic lower phase, which was discarded. The aqueous phase was added with isopropanol (600 µl) and, after agitation, the samples were incubated at -20°C for at least 60 min and centrifuged at 15,000xg at 4°C for 5 min. The supernatant was discarded. The pellet was washed with ethanol at 80% and then with absolute ethanol, concentrated to dryness and suspended again in water (20 µl). 2 µl of the sample were used for the spectrophotometric determination of RNA concentration at the wave length of 260 nm and for the visualization with ethidium bromide on agarose gel at 1%.

Reverse transcription and amplification by Polymerase Chain Reaction (PCR) of CB2 receptor

1 µg of total RNA withdrawn from each of the above samples was subjected to reverse transcription, as described by Leon at al. (PNAS, 91:3739-3743, 1994), using the reverse transcriptase of murine moloney leukaemic virus and 50 pmol of the synthetic oligonucleotide SEQ ID NO:1, having sequence 5'-TAGGTAGGAGATCAAGCG-3', complementary and antiparallel to the mRNA coding for cannabinoid peripheral receptor (Munro et al., Nature 365, 61- 65, 1993), in a total volume of 20 µl. After 1-hr reaction at 37°C, the volume of the transcription product was brought to 100 µl with water and one fourth of the final volume was subjected to amplification by means of PCR. Said operation was conducted in 500 µl thin-walled test tubes, filled up to a final volume of 100 µl with a solution having the following composition: 50 mM TRIS HCl, pH 8.3, at 25°C:

75 mM KCl;
2.5 mM $MgCl_2$;
10 mM DTT (dithiothreitol);
0.2 mM dATP, dCTP, dGTP and dTTP;
50 pmol sense primer SEQ ID NO:2 (5'-TTTCACGGTGTGGACTCC-3');
50 pmol antisense primer SEQ ID NO:3 (5'-TAGGTAGGAGATCAAGCG-3');
0.551 *Taq* Pol (Taq polymerase) 2.5 U/µl (Polymerase Stoffel fragment, Cetus/Perkin-Elmer®);
3% formamide.

Primers were specific for cannabinoid peripheral receptor. PCR was carried out for 35 thermal cycles in apparatus mod. 9600 of Cetus/Perkin Elmer®, according to the following procedure:

| Temperature | Duration |
|---|---|
| 95°C | 1 min |
| 54°C | 1 min |
| 72°C | 1 min |

Once the reaction had been completed, 30 µl of the reaction volume was electrophoresed on 1% agarose gel

in order to visualize the amplification products, then transferred and immobilized on a nylon filter for inner-probe hybridization to the two PCR primers.

Specific hybridization with inner oligonucleotide

The identity of the amplification product was determined by hybridization with a synthetic oligonucleotide SEQ ID NO:4, complementary to amplified sequences of cannabinoid peripheral receptor, labelled with radioactive tracers.
The sequence SEQ ID NO:4 was as follows:

5'-GGTGACGAGAGCTTTGTAGGTAGGTGGGTAGCACAGACATAGGTA-3'

Radioactive labelling was carried out at 37°C for 1 hr, using 5 pmol inner oligonucleotide, as follows: 33 pmol ($\alpha^{32}P$)-dATP (New England Nuclear);
20 U Terminal Deoxynucleotide Transferase (US Biochemical);
100 mM sodium cacodylate (pH 7.2)
2 mM $CoCl_2$
0.2 mM 2-mercaptoethanol in a final volume of 20 µl.

The labelled products were purified by chromatography on Sephadex G-50® column, and added to the hybridization solution containing the filter with the PCR-amplified products. Hybridization and washings were carried out under standard conditions, as described by Sambrook, Fritsch and Maniatis (Molecular Cloning. A Laboratory Manual, Cold Spring Harbor Laboratory Press, 1989).
The hybridized filter was exposed to a autoradiographic film for the detection of the amplified bands of the cannabinoid peripheral receptor messenger.

**ii) m-RNA - Peripheral Cannabinoid Receptor in situ hybridisation.**

The brains of adult male mice (20-22 g) were frozen in 2-methyl-butane at -80°C, sectioned with a cryostat and the 12 mm coronal sections were thaw-mounted on poly-L-lysine coated slides. All sections were then fixed in 4% paraformaldehyde (PFA) and dehydrated in a graded series of ethanol, including a 5 min. incubation in chloroform and air dried. Mouse cerebellar granule cells (15 day in vitro-DIV) cultured on poly-L-lysine coated coverslips were fixed in 4% PFA, washed twice in PBS and permeabilized in 70% ethanol at 4°C for 48 hrs, dehydrated in higher graded ethanol and air dried. To detect the CB2-specific mRNA the synthetic oligonucleotide (45 mers) CB2 (SEQ ID NO:4: 5'-GGTGACGAGAGCTTTGTAGGTAGGTGGGTAGCACAGACATAGGTA-3 according to Munro et al., 1993, cited reference) was chosen.
A random sequence was utilized for control sections. All oligonucleotides were tailed with $^{35}S$-dATP (NEN) using terminal deoxynucleotidyl-transferase (Pharmacia) to a specific activity of $10^9$ cpm/mg. All sections were hybridized in standard solutions (*) with 1.5 107 dpm/ml overnight at 42°C in a humidified chamber. Sections were then washed once in 1X SSC/0.1%(**) SDS at 55°C for 30 min., then twice in 1X SSC at 55°C for 15 min., followed by 0.1X SSC at 25°C for 30 min., rinsed in autoclaved water for 2min., dehydrated in ethanol and air dried. The slides were subsequently dipped in photoemulsion (Ilford K.5 diluted 1:1 in water), exposed for 5 weeks at 4°C, developed (Ilford Phenisol), fixed (Ilford Hypam) and counterstained with cresyl violet.

Standard Solution preparation

(*) Hybridisation cocktail Formamide: 5 ml, 50%; SSC 20x: 2 ml, 4 x; Denhardt's 50x: 0.2 ml; 1x Lauryl-sacarosyl 20%: 0.5 ml, 1%; Destran sulphate: 1 gr, 10% ; 200 mM Phosphate buffer pH 7.0: 1 ml, 20 mM, water-DEPC q.s. to 10 ml. Filtrate using 0.45 µm filter and store at -20°C .
(**) Sodium Citrate Solution SCS 20x. Sodium chloride 175.3g and sodium citrate 88.2g were dissolved in 800ml DEPC-water; NaOH 10M was used to adjust the pH 7.2. Add DEPC-water q.s. to 1000ml for autoclave procedure.
The autoradiographic examination shows that rat peritoneal mast cells and the cousin RBL-2H3 cells, cerebellar granule cells and cerebellum express, similarly to spleen preparations, mRNA specific to peripheral cannabinoid receptor CB2. This finding is clearly shown in Figures 1 and 2. Figure 1 illustrates the specific hybridization of cannabinoid peripheral receptor amplified by Polymerase Chain Reaction (PCR) respectively in (A) rat spleen, (B) rat peritoneal mast cell cultures and (C) RBL-2H3 cell cultures (C). Figure 2 illustrates the specific mRNA-CB2 peripheral cannabinoid receptor in situ hybridisation.

**B) Binding assays**

Specific binding assays were conducted for the purpose of checking whether the receptor was functionally expressed and whether the amides forming the object of the present invention were able to bind to CB2 receptor in a specific and competitive manner, compared with the cannabinoids having a known affinity to the receptor and compared with anandamide, the endogenous ligand of CB1 receptor.

a) Preparation of plasmatic membranes of RBL-2H3 cells

RBL-2H3 cells (100 x $10^6$), frozen at -80°C, were thawed with 4 ml of Tris-HCl buffer solution 50 mM (pH 7.4), added with 0.25% w/v of Tripsin inhibitor, type II-S: soy bean® (distributed by Sigma). The cells were resuspended in the buffer and homogenized. The homogenate was centrifuged at 1500xg at 4°C for 10 min. The supernatant obtained was collected and the precipitate was resuspended in 4 ml of the previous buffer. The resuspended precipitate was homogenized and centrifuged again at 1500xg at 4°C for 5-10 min. The resulting supernatant was combined to the supernatant previously obtained and centrifuged at 5000xg for 10 min. After centrifugation, the supernatant was collected and further contrifuged at 40,000xg at 4°C for 30 min. The resulting precipitate was resuspended in 0.5 ml of the buffer solution described above, with addition of 1% Bovine Serum Albumin fatty acid free. The obtained suspension was centrifuged at 40,000xg at 4°C for 30 min. The resulting precipitate was collected and resuspended in buffer solution containing 50 mM Tris HCl, 1 mM Tris-EDTA, 3 mM $MgCl_2$, pH 7.4, in order to obtain a proteic concentration of 1μg/μl approx. This preparation may be used fresh, or frozen at -80°C and utilized within few days.

b) "Binding" assays conditions to the preparation of RBL-2H3 cell membranes

Saturation curve of labelled receptor agonist 3H-WIN 55,212-2 (specific activity 44 Ci/mmol, distributed by New England Nuclear): silanized plastic test tubes were fed, in the order, with binding buffer (50 mM Tris HCl, 1 mM Tris-EDTA, 3 mM $MgCl_2$, 0.5% w/v Bovine Serum Albumin fatty acid-free) to a final volume of 0.5 ml, decreasing doses of 3H-WIN 55,212-2 from 0.5 to 20 nM (final) and 30 μg membrane proteins prepared in item a). The resulting binding mixture was incubated at 30°C for 60 min under stirring and centrifuged at 40,000xg at 20°C for 15 min. After centrifugation, an aliquot portion of the supernatant was collected to calculate the concentration of the ligand not associated to the membranes. After removal of the supernatant residue, the precipitate was washed with 1 ml PBS (Phosphate Buffer Solution) containing 0.5% Bovine Serum Albumin, taken up with 50 μl of a mixture of ethanol and 1% Triton X-100® (50/50 v/v), incubated at 37°C for 20 min and resuspended.

The resuspended material was added and mixed with scintillator liquid (3 ml) and placed in a "β-counter" for 5 min. To evaluate the "Aspecific Binding", the predetermined test tubes were fed first with unlabelled receptor agonist WIN 55,212-2 at a final concentration of 15 M and then with the labelled ligand. Said binding gives Kd = 5-10 nM and Bmax = 100-250 pM.

3H-WIN 55,212-2 binding displacement

For these competitions, 3H-WIN 55,212-2 was used at a concentration of 3 μM; non-radioactive WIN 55,212-2 (1μM) was used to inhibit specific binding. The competitors Nabilone [3-(1,1-dimethylheptyl)-6,6a,7,8,10,10a-hexahydro-1-hydroxy-6,6-dimethyl-9H-dibenzo-[b,d]-pyran-9-one] and anandamide were solubilized in 0.1% ethanol, whereas the products forming the object of the present invention were solubilized in 0.1% DMSO. The competitors were added to the binding mixture prior to the labelled ligand. In both cases, for an evaluation of the reference specific binding, ethanol and DMSO were added at the same final concentration to the total binding and to the aspecific one, also in the absence of competitors.

The aforementioned binding assays provided evidence that the receptor expression was complete and functional.

The data of Table 1 show that the amidic derivatives according to the present invention, like anandamide and the cannabinoid Nabilone, are able to bind to the receptor and to compete, with different potency, with the synthetic radioligand WIN 55,212-2.

Table 1 -

| Competitive inhibition of I3HI WIN 55,212-2 specific binding by the compounds of the invention. I3HI WIN 55,212-2 was used at a concentration of 3 µM, whereas the other compounds were used at a concentration of 10 µM. | | |
|---|---|---|
| Competitor | Specific binding | Displacement (%) |
| 0.1% DMSO* | 1433 | - |
| Example 1 | - | 100 |
| Example 7 | 297 | 79 |
| 0.1% Ethanol** | 1843 | - |
| Anandamide | 99 | 95 |
| Nabilone | - | 100 |

* 01.% DMSO is the concentration used for amides solubilization according to the present invention.
**0.1% ethanol is the concentration used for Anandamide and Nabilone solubilization.

Table 2 -

| Competitive inhibition of I3HI WIN 55,212-2 binding to RBL-2H3 cell membranes by the amides of the invention and by the cannabinoids. Incubation was carried out in the presence of 1% DMSO. Inhibitory Concentration 50 values ($IC_{50}$) are expressed as means ± SEM, after the number of experiments indicated. | | |
|---|---|---|
| Compound | $IC_{50}$ (nM) | no. of experiments |
| Nabilone | 2.6±1.4 | 4 |
| Δ 8-THC | 223±120 | 6 |
| Cannabidiol | > 1000 | 3 |
| Anandamide | 33±29 | 5 |
| Example 1 | 0.04;0.05 | 2 |
| Example 7 | 0.16;0.005 | 2 |
| Example 14 | 8.9±.4.8 | 4 |

**C) Biological activity assays**

The biological activity deriving from CB2 receptor functional activation was evaluated in RBL-2H3 cultures and in mouse cerebellar granule cell primary cultures. In particular, the ability of the amides of the invention to inhibit serotonin release, induced by specific stimulus, was evaluated on RBL cultures and compared with cannabinoids of known potency and with anandamide.

The specific binding and/or the functional effects of the amidic derivatives described in Examples 1, 2, 5-11 and 13-18 were evaluated. For purpose of comparison, the following products were used: among natural cannabinoids, three different isomers of tetrahydrocannabinol (THC): Δ 8-THC, Δ 9-THC and 11-nor-Δ 8-THC-9 ; among synthetic cannabinoids, Nabilone and WIN 55,212-2, in addition to the CB1 endogenous ligand anandamide. Furthermore, it was shown that CB2 receptor plays a role in protecting against Excitatory Amino Acid (EAA)-induced cell death.

Preparation of RBL-2H3 cell cultures

RBL-2H3 cell cultures were grown in flasks (Falcon®), at 37°C, 5% $CO_2$, in the presence of Minimum Essential Eagle's Medium (MEM) and 4 mM glutamine, 100 U/ml penicillin and 20% deactivated fetal calf serum (DFCS).

RBL-2H3 cell sensitization and activation

For each test, the cells were removed from the flasks with PBS, containing 0.5 mM EDTA at pH 7.2, and seeded in wells (96 well plate Falcon ®) in the presence of RPMI-1640 medium (code 6504, distributed by Sigma) containing 50 mg/l gentamicin and 10% FCS at a density of 100,000 cells/100 µl medium/well. Furthermore, during seeding, said cells were loaded with serotonin by addition of 1 µCi/ml 3H-serotonin (5-hydroxy-triptamine 5HT, 26.4 Ci/mmol, New England Nuclear) and then incubated for 18 hrs (37°C, 5% $CO_2$). After 18-hr incubation in the presence of 3H-serotonin, the culture medium was removed and the cells were sensitized for 1 hr (37°C, 5% $CO_2$) in the presence of PIPES (N,N'-bis-(2-ethanesulphonyl)-piperazine, code 3768, Sigma) -buffered saline (100 µl/well), pH 7.1, contain-

ing 0.3 μg/ml mouse monoclonal antibody (IgE) against DNP (ADNP).

After removal of the sensitization medium, cells were activated - or not activated (control) - by addition, at 37°C for 155 min, of PIPES (100 μl/well), pH 7.1, containing - or not containing (control) - 0.1 μg/ml human albumin combined with dinitrophenyl (DNP-HSA) and containing a natural or synthetic cannabinoid or an amidic derivative according to the present invention. After incubation, the medium was collected and centrifuged to determine the 3H-serotonin quantity released from DNP-HSA-stimulated or not stimulated cells. In parallel, adhered cells were solubilized with 1% Triton X-100® in PBS (100 μl/well) in order to determine the quantity of 3H-serotonin present in the cells. In both cases. the quantity of 3H-serotonin was measured by liquid scintillography and radioactivity counting, using a β-counter of Canberra Packard, 1900 TR®.

Addition of cannabinoids or of the derivatives according to the present invention during RBL-2H3 cells activation

It was evaluated the effect of the amidic derivatives of the invention (stock solution in DMSO) and of cannabinoids in the presence or in the absence of anandamide (stock solution in ethanol) on DNP-HSA-induced 3H-serotonin release in DNP sensitized cells. To this purpose, cannabinoids or the amidic derivatives of the invention, with or without addition of anandamide, were added to the cell activation medium (PIPES ± DNP-HSA) at the desired concentration. In all cases, the medium was incubated at 37°C for 15 min, while the total final concentration was kept constant (0.2% solvents).

The amides of the invention were solubilized in 0.2% DMSO and 0.1% absolute ethanol, except for N-palmitoyl-L-serine, which was solubilized in 1% DMSO and 0.1% ethanol.

Quantification of 3H-serotonin net release after RBL-2H3 cells activation

3H-serotonin release in the various samples was calculated according to the following formula:

$$\text{release} = \frac{\text{dpm released}}{(\text{dpm released} + \text{dpm associated with the cells})} \times 100$$

where "dpm" means nuclear disintegrations per minute.

The effect of the amides of the invention was expressed as the percentage of 3H-serotonin net release (i.e. after deduction of the percentage of release in the absence of DNP-HSA) or as the percentage of 3H-serotonin release inhibition.

The results of evaluation tests on the functional effects of cannabinoids and of the amidic derivatives of the invention are shown in Table 3. These data indicate that natural cannabinoids Δ8-tetrahydrocannabinol (Δ8-THC) and Δ9-tetrahydrocannabinol (Δ9-THC), the synthetic cannabinoid Nabilone and the labelled receptor agonist WIN 55,212-2 are able to inhibit, in a concentration-dependent way, RBL cells immunogenic activation measured as serotonin release. It is evident that the compounds described in Examples 1 and 7 can inhibit the effects induced by mast cell activation and exert an effect that is higher than or comparable to the one of cannabinoids. Conversely, anandamide is unable to affect serotonin release, thus demonstrating a specific structural selectivity in the receptor functional activation.

The compounds derived from saturated carboxylic acids are more potent in exerting the functional effects following receptor activation and this is confirmed by the comparative tests results, reported below, wherein it is reported the effect of N-(2-hydroxyethyl)hexadecanamide, wherein the acyl-moiety is sature, and that of N-(2-hydroxyethyl)-lnoleylamide, wherein the acyl-moiety presents two double bonds.

Table 3 -

| Effect of natural and synthetic cannabinoids and of the amides according to the present invention on serotonin release induced by ADNP/DNP.HSA immunogenic stimulation in RBL-2H3 cells. The tested compounds were solubilized in 0.1% DMSO and 0.1% ethanol, a part from N-(2-hydroxyethyl)-hexadecanamide which was solubilized in 1% DMSO and 1% ethanol. | | |
|---|---|---|
| Molecules added after sensitization with ADNP | 3H-serotonin net release (%) | ED50 (μm) |
| DNP.HSA- | 100 | |
| DNP.HSA+WIN 55,212-2 (50μM) | 0 | 6.4 |
| DNP.HSA+Nabilone (10μM ) | 0 | 2.8 |
| DNA.HSA+ Δ8-THC (25μM ) | 0 | 5 |
| DNP.HSA+Δ9-THC (25μM) | 27 | 5 |

Table 3 - (continued)

| Effect of natural and synthetic cannabinoids and of the amides according to the present invention on serotonin release induced by ADNP/DNP.HSA immunogenic stimulation in RBL-2H3 cells. The tested compounds were solubilized in 0.1% DMSO and 0.1% ethanol, a part from N-(2-hydroxyethyl)-hexadecanamide which was solubilized in 1% DMSO and 1% ethanol. | | |
|---|---|---|
| Molecules added after sensitization with ADNP | 3H-serotonin net release (%) | ED50 (μm) |
| DNP.HSA+11-nor-Δ8-THC-9 (20μM) | 33 | 15 |
| DNP.HSA+ anandamide | 100 | - |
| DNP.HSA+N-(2-hydroxyethyl)--hexadecanamide (100μM ) | 0 | 2.6 |
| DNP.HSA+N-(2-hydroxyethyl)--linoleylamide (100μM) | 100 | - |
| DNP.HSA+Example 1 (100μM ) | 0 | - |
| DNP.HSA+Example 7 (10μM ) | 27 | 4.8 |

Co-incubation of anandamide with natural or synthetic cannabinoids and with the derivative described in Example 7 reduces the ability of the acyl amide of the invention, as well as of cannabinoids, to inhibit serotonin release, as reported in Table 4.

Table 4 -

| Anandamide antagonism towards the inhibitory effect of cannabinoids and amidic derivatives of the invention on ADNP/DNP.HSA-induced activation in RBL-2H3 cells. | | |
|---|---|---|
| Molecules added after sensitization with ADNP | 3H-serotonin net release inhibition (%) | |
| | -anandamide | + anandamide (12.5 μM) |
| DNP.HSA+WIN 55,212-2 (30μM) | 100 | 36 |
| DNP.HSA+Nabilone (5μM) | 90 | 50 |
| DNP.HSA+ Δ8-THC (25μM) | 100 | n.d. |
| DNA.HSA+Δ9-THC (25μM) | 73 | n.d. |
| DNA.HSA+11-nor-Δ8-THC-9 (20μM ) | 67 | n.d. |
| DNP.HSA+Example 7 (10μM) | 73 | 24 |

The data reported in Table 5 show that the co-incubation of anandamide with natural or synthetic cannabinoids and with the amidic derivatives according to the present invention reduces the potency of anandamide, indicating that anandamide causes a competitive partial antagonism on the peripheral receptor. Therefore, as shown in Table 1, anandamide, though having binding affinity for the peripheral receptor, is not able to induce biological effects and, by competing for the receptor, functionally antogonizes the protective effects of cannabinoids.

Table 5 -

| $ED_{50}$ of cannabinoids and of the amidic derivatives of the invention on the inhibition of 3H-serotonin release induced by ADNP/DNP.HSA stimulation in RBL-2H3 cells, in the presence and in the absence of anandamide. | | |
|---|---|---|
| Molecules | ED50 (μM) | |
| | -anandamide | +anandamide (12.5 μM) |
| WIN 55,212-2 | 6.4 | 40 |
| Nabilone | 2.8 | 5 |
| Δ8-THC | 5 | n.d. |
| Δ9-THC | 5 | n.d. |
| 11-nor-Δ8-THC-9 | 15 | n.d. |
| Example 7 | 4.8 | >100 |

Table 5 - (continued)

| ED$_{50}$ of cannabinoids and of the amidic derivatives of the invention on the inhibition of 3H-serotonin release induced by ADNP/DNP.HSA stimulation in RBL-2H3 cells, in the presence and in the absence of anandamide. | | |
|---|---|---|
| Molecules | ED50 (μM) | |
| | -anandamide | +anandamide (12.5 μM) |
| Example 14 | 0.51 | 3.30 |
| Example 16 | 2.90 | 4.20 |

The partial competitive antagonism of anandamide on the peripheral receptor is exerted by the integral molecule and not by anandamide metabolites, as shown in Table 6.

Table 6 -

| 3H-serotonin release inhibition by WIN 55,212-2 (used at a concentration of 30 μm): antagonistic effect of anandamide vs. the metabolites of the same. | | | |
|---|---|---|---|
| 3H-serotonin net release inhibition (%) | | | |
| | | -WIN | +WIN |
| DNP.HSA | | 0 | 69 |
| DNP.HSA | + 12.5μM anandamide | 7 | 15 |
| DNP.HSA | + 12.5μM ethanolamine | 6 | 72 |
| DNP.HSA | + 12.5μM arachidonic acid | 4 | 59 |
| DNP.HSA | + 12.5μM ethanolamide<br>+ 12.5μM arachidonic acid | 3 | 61 |

The tests results reported in Tables 4, 7 and 8 show that the receptorial specificity and the level of activity are functional to the nature of both the acylic and the amidic moieties of the amidic derivatives according to the present invention.

Table 7 -

| Inhibitory effect of the amidic derivatives according to the present invention on 3H-serotonin release induced by ADP/DPN.HSA immunogenic stimulation in RBL-2H3 cells. | | |
|---|---|---|
| Compounds | 3H-serotonin release inhibition % | |
| | DMSO 0.2% | DMSO 1% |
| Example 2 | 79 (100μM ) | 61 (100μM ) |
| Example 5 | | 61 (100μM) |
| Example 6 | 50 ( 60μM) | 91 (100μM) |
| Example 7 | 85 ( 60μM) | 100 (100μM) |
| Example 8 | 93 (100μM ) | 100 (100μM) |
| Example 9 | 55 (100μM) | |
| Example 10 | 47 (100μM) | |
| Example 13 | | 54 (100μM) |
| Example 14 | 76 ( 20μM) | 91 (100μM) |
| Example 15 | 67 ( 20μM) | 86 (100μM) |
| Example 16 | 92 ( 20μM) | 86 (100μM) |

Numbers in paretheses are the concentrations (μM) used for the various compounds in the different tests.

Table 8 -

| Inhibition of I3HI serotonine release from DNP-HSA activated RBL-2H3 cells. The compounds of the invention were present during the 15 minutes release period. EC50 is the concentration inhibiting by 50% the net release of I3HI serotonin from DNP-HSA - activated RBL-2H3 cells. The reported values are expressed as means ± SEM, after the number of experiments shown in parentheses. | | |
|---|---|---|
| Compound | EC50 | |
| | 0.2% DMSO | 1% DMSO |
| Example 5 | 104 ± 59 (2) | |
| Example 6 | | 29 ± 12 (3) |
| Example 8 | 10 ± 1.5 (3) | 7 ± 5 (2) |
| Example 10 | | 30 (1) |
| Example 11 | 26 ± 18 (4) | |
| Example 14 | 1.28±0.38 (6) | 0,12±0.07 (3) |
| Example 15 | 12 (1) | |
| Example 16 | 2.4 ± 0.5 (4) | |

Cannabinoid neuroprotective effect in cerebellar granule cell culture

Granular cell cultures were obtained from postnatal day 8-9 mouse Balb-6 cerebellum. The cells were suspended in EBM + 2mM L-glutamine, 100 U/ml penicillin 50µg/ml gentamycin, 25mM KCl and 10% fetal calf serum, plated on polylisine substrate, cultured from 8-10 days and then exposed to glutamate (500 µM) for 5 minutes at room temperature, exiting in 60% cell death. Cannabinoids and anandamide were solubilized in Locke's solution and added to the culture for 10 minutes, starting 15 minutes after removing glutamate. The number of survived cells was calculated by the colorimetric method MTT, 24 hours after the washing out of thr compound.

Cannabinoids protect against cell death induced by glutamate. To the contrary, anandamide, which can not functionally activate CB2 cannabinoid peripheral receptor, does not display any protective effect. as shown in Table 9; anadamide was not effective up to 100 µM. Furthermore, the data reported in Table 9 show that CB2 receptor is functionally expressed also on neurons culture. being able to play a protective role on cell survival; this indicates that CB2 receptor capability of modulating events is non-directly mediated by immune lineage cell.

Table 9 -

| Cannabinoid neuroprotective effect in comparison with anandamide. The reported values are expressed as means ± SEM, after the number of experiments indicated in parentheses. | |
|---|---|
| Compound | EC50 |
| Nabilone | 3.90 ± 1.1 (3) |
| Anandamide | no effect (3) |

The experimental data reported above prove a precise structureactivity correlation in the selective activation of CB2 peripheral receptor, which mediates the non-psychoactive effects of cannabinoids. More precisely, the molecules having a saturated acylic chain are able to bind and, above all, to functionally activate CB2 peripheral receptor to a significant extent. To the contrary, compounds with more than one double bond in the acyl chain, though having affinity to CB2 receptor, are not able to activate said receptor functionally, thus acting as partial competitive receptor antagonists.

Furthermore, the aforementioned experimental results demonstrate that mast cells express the cannabinoid peripheral receptor and that the amidic derivatives according to the present invention behave as competitive agonists of CB2 receptor with an affinity that is higher than, or in any case comparable with the one of natural and synthetic cannabinoids. Moreover, they functionally activate said receptor. Importantly, the Applicant has found that also non-immune cells functionally express the cannabinoid peripheral receptor CB2, which for example was observed in cerebellar granule cells, cerebellum, heart and lung.

Said results are extremely interesting from a therapeutic point of view: in fact, the amides of the invention are suitable for the treatment of all diseases for which cannabinoids are notoriously efficacious, acting only on CB2 peripheral receptor and excluding the activity mediated by CB1 central receptor, responsible for untoward effects.

It is also very important the fact that the amidic derivatives according to the invention can directly or indirectly

regulate the defence response to various noxae involving both cell types belonging to the immune lineage and neurons or cells having different physiological function, i.e. heart and lung, by modulating mast cell degranulation through the selective activation of CB2 receptor. The importance of mast cells in inflammatory processes connected with several acute and chronic pathologies is well known in the state of the art (Otten U. et al., Nerve Growth Factor induces growth and differentiation of human B lymphocytes, PNAS, 86:10059-10063, 1989; Marshall J. S. et al., 1989, The J. of Immunology, 144:1866-92; B: Henderson and S. Blake, Therapeutic potential of cytokine manipulation, TIPS, 13,145-152, 1992; M.C.Morganti-Kossmann et al., Cytokines and neuropathology, TIPS, 13, 286-290, 1992; A.S. Fauci, Science, 262, 1011- 1018, 1993; E.A. Formukong at al., Analgesic and Antiinflammatory Activity of Constituents of Cannabis Sativa L., Inflammation, 12, 4:361- 371, 1988; W.D. Lyman. Drugs of Abuse and Experimental Autoimmune Diseases, Exp. Med. Biology, 288:81-82, 1991; G.H. Gibbons et al., The Emerging Concept of Vascular Remodeling, Mechanisms of Disease, 330. 20: 1431-1438, 1994; P.T. Kovanen, The mast cell, a potential link between inflammation and cellular cholesterol deposition in atherogenesis, Europen Heart Journal, 14 suppl. K: 105-117, 1993).

In other words, thanks to their specific characteristics, the amidic derivatives of the invention are able to selectively bind and to functionally activate CB2 cannabinoid peripheral receptor. Therefore, they represent an important therapeutic tool both for the treatment of pathologies connected with the modulation of CB2 cannabinoid peripheral receptor and for the treatment of the pathologies deriving, for example, form mast cell degranulation influenced by CB2 receptor.

Therefore, the therapeutic use of said compounds is particularly useful in the treatment of diseases connected with an anomalous modulation of CB2 peripheral receptor, or that benefit of the activation of said receptor with consequent negative modulation of cytotoxic or proinflammatory phenomena, connected with cytokine, neurokine or enzyme release, or with second messenger activation, such as:

- pathologies connected with the immune system alteration;
- pathologies with etiologic autoimmune component, such as multiple sclerosis, lateral amyothrophic sclerosis, and the associated muscolar spasm;
- articular acute and chronic inflammatory pathologies, also on autoimmune basis, such as rheumatoid arthritis;
- pathologies caused by biological agents such as viral (HIV) and bacterial encephalitic meningitis, bacterial meningitis, meningitis by cytomegalovirus and AIDS-dementia complex;
- chronic neurodegenerative pathologies, such as senile dementia, Alzhaeimer's and Parkinson's diseases, and the associated cachectic symptomatology;
- pathologies with nociception alteration;
- pathologies associated with EAA excitotoxicity such as cerebral ictus, TIA, cerebral and spinal cord trauma, epilepsy, migraine and chorea;
- cardiovascular pathologies associated with vascular remodelling i.e. restenosis after angioplasty including stent application, atherosclerosis and heart attack;
- pathologies involving pressure alterations (hypertension) at cardiovascular, pulmonary and ocular levels, i.e. glaucoma;
- chronic airway obstruction, including asthma;
- nausea, also of hyatrogenic nature.

In view of their effects, the amidic derivatives according to the present invention are suitable for the treatment of human and animal diseases.

For all aforesaid diseases, the systemic administration of the claimed compounds by the oral or parenteral or topical or transdermic ways may be envisaged.

The therapeutically effective dose varies depending on the way of administration, as well as on the disease seriousness; it also varies depending on the patient's age, weight and general health conditions. In any case, acceptable therapeutic doses may range from 0.1 to 20 mg/kg/die, over variable periods, in any case for at least 30 days. The pharmaceutical compositions containing as the active principles the amides according to the present invention are inclusive of all formulations containing pharmaceutically acceptable excipients, that are suitable for the administration of the active ingredients in the forms best suited to the disease to be treated and, in any case, rendering the active ingredient as bioavailable as possible. In particular, solutions or suspensions for general i.v., s.c. and i.m. administration, solutions for ophthalmic treatment in the form of eyewash, solid or semisolid formulations in the form of inserts, gels and ointments are to be envisaged. As concerns oral formulations, granular powders, tablets, pills and capsules will be preferred. As concerns dermic and transdermic administration, creams, ointments, gels and plasters, where the active ingredient may be included in slow-releasing microspheres, will be preferred.

| EXAMPLE 19 | |
|---|---|
| Vials for injection | |
| Every vial contains: | |
| Compound of Example 8 | 20 mg |
| Mannitol | 12 mg |
| Sodium metasulphite | 1.3 mg |
| Benzyl alcohol | 80 mg |
| Propylene glycol | 400 mg |
| Sodium hydroxide | q.s. to pH 7 |
| Water for injectable formulations | q.s. to 2 ml |
| **EXAMPLE 20** | |
| Tablets | |
| Every tablet contains: | |
| Compound of Example 1 | 50 mg |
| Dibasic dihydrated calcium phosphate | 135.2 mg |
| Microgranular cellulose | 36 mg |
| Maize starch | 7.2 mg |
| Magnesium stearate | 1.8 mg |
| Hydrogenated vegetable oil | 1.2 mg |
| Precipitated silica | 0.6 mg |
| Hydroxypropylene ethylcellulose | 4.7 mg |
| Titanium dioxide | 0.3 mg |
| **EXAMPLE 21** | |
| Eyewash | |
| Every bottle contains: | |
| Compound as per Example 7 | 25 mg |
| Borax | 15 mg |
| Boric acid | 75 mg |
| Polysorbate 80 | 15 mg |
| Lactose | 80 mg |
| Phenol | 3.9 mg |
| Disodium edetate | 5 mg |
| Water for injectable formulation | q.s. to 5 ml |
| **EXAMPLE 22** | |
| Soft capsules | |
| Every capsule contains: | |
| Compound of Example 16 | 100 mg |
| Excipient: peanut oil O.P. | 100 mg |
| Composition of the capsule: | |
| gelatin O.P., glycerin O.P., natural dye E12 | |

## Claims

1. Amide of formula (I)

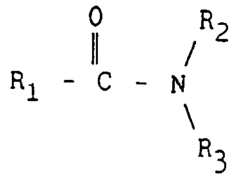

(I)

wherein $R_1$ can be:

1) a linear or branched hydrocarbon radical having from 9 to 23 carbon atoms, saturated or presenting one double bond, optionally substituted with one or more -OH groups;
2) a group of formula (II):

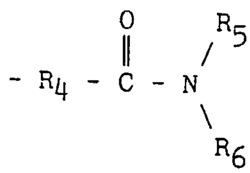

(II)

wherein $R_4$ is a linear hydrocarbon radical, saturated or containing one double bond, comprising from 7 to 22 carbon atoms, optionally substituted with one or more linear or branched alkyl groups $C_1$-$C_8$ and/or with one or more -OH groups;
3) a group of formula (IV):

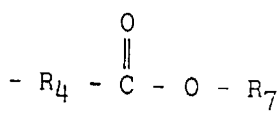

(IV)

wherein $R_4$ has the meanings described above and $R_7$ = -H or a linear or branched alkyl group, comprising from 1 to 20 carbon atoms; the residues of formulae:

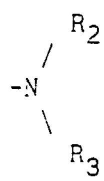

(III) and

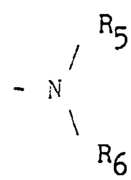

(V)

equal or different from each other, are glycosaminic residues of formula:

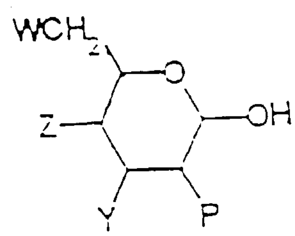

wherein one of the groups P, Y and Z is

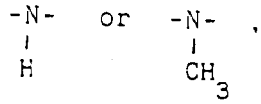

and the remaining groups, including W are -H or -OH; the residues $R_2$, $R_3$, $R_5$ and/or $R_6$ of formulae (III) and (V) being optionally substituted with at least a pharmaceutically acceptable radical capable of increasing the solubility of the amide of formula (I) said radical being selected from acyls, hemiacyls of dicarboxylic acids, dialkylaminoacyls alkyl sulphonates, alkyls, O-phospates and O- sulphates; with the proviso that said amide of formula (I) is different from 2-deoxy-2-[(3-hydroxy-1-oxotetradecyl)-amino]-α-D-glucopyranose.

**2.** The amide according to claim 1, characterised in that, when $R_1$ belongs to class (1), it is a radical having from 11 to 17 carbon atoms.

**3.** The amide according to claim 1, characterised in that, when $R_1$ belongs to class (2), $R_4$ is a radical containing from 10 to 16 carbon atoms.

**4.** The amide according to claim 1, characterised in that said acyl is acetyl or benzoyl.

**5.** The amide according to claim 1, characterised in that said emiacyl of a dicarboxylic acid is the emiacyl of succinic or glutaric acid.

**6.** The amide according to claim 1, characterised in that said alkylsulphonate is metansulphonate or p-toluen-sulphonate.

**7.** The amide according to claim 1, characterised in that said alkyl is methyl or ethyl.

**8.** The amide according to claim 1, characterised in that, when $R_1$ belongs to class (1), it forms, together with the adjacent carbonyls, the acyl group of a monocarboxylic acid selected from the group consisting of lauric acid, myristic acid, palmitic acid, stearic acid, palmitoleic acid, oleic acid and ω-hydroxy-palmitic acid.

**9.** The amide according to claim 1, characterised in that, when $R_1$ belongs to classes (2) and (3), $R_4$ forms with the two adjacent carbonyls the acyl group of traumatic acid.

**10.** The amide according to claim 1, characterised in that said glycosamine is selected from the group consisting of D-Glucosamine, L-Acosamine, D- Mannosamine and D-Galactosamine.

**11.** Process for the preparation of amides of formula (I):

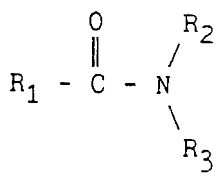

(I)

wherein $R_1$ can be:

1) a linear or branched hydrocarbon radical having from 9 to 23 carbon atoms, saturated or presenting one double bond, optionally substituted with one or more -OH groups;

2) a group of formula (II):

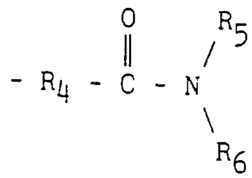

(II)

wherein $R_4$ is a linear hydrocarbon radical, saturated or containing one double bond, comprising from 7 to 22 carbon atoms, optionally substituted with one or more linear or branched alkyl groups $C_1$-$C_8$ and/or it one or more -OH groups;

3) a group of formula (IV):

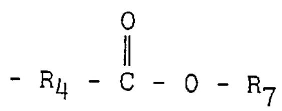

(IV)

wherein $R_4$ has the meanings described above and $R_7$ = -H or a linear or branched alkyl group, comprising from 1 to 20 carbon atoms; the residues of formulae

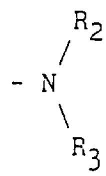

(III) and

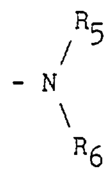

(V)

equal or different from each other, are glycosaminic residues of formula:

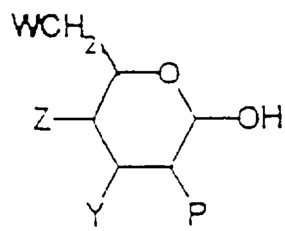

wherein one of the groups P, Y and Z is

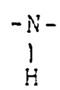

or

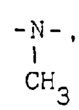

,

and the remaining groups, including W, are -H or -OH; the residues $R_2$, $R_3$, $R_5$ and/or $R_6$ of formulae (III) and (V) being optionally substituted with at least a pharmaceutically acceptable radical capable of increasing the solubility of the amide of formula (I)said radical being selected from acyls, hemiacyls of dicarboxylic acids, dialkylaminoacyls alkyl sulphonates, alkyls, O-phosphates and O-sulphates; with the proviso that said amide of formula (I) is different from 2-deoxy-2-[(3-hydroxy-1-oxotetradecyl)-amino]-α-D-glucopyranose; comprising the reaction of an activated form of the acid $R_1$-COOH, of formula $R_1$-CO-X, with an amine of formula

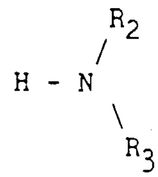

in aqueous or organic solvents in the presence of a base, under stirring at a temperature ranging from -20° to + 60°C, for a period ranging from 10 minutes to 24 hours; the optional acidification of the reaction medium; the isolation and the purification by conventional techniques.

**12.** The process according to claim 11, characterised in that said temperature is comprised between -10° and 20°C.

**13.** The process according to claim 11, characterised in that the reaction time is comprised between 1 hour and 3 hours.

**14.** The process according to claim 11, characterised in that said activated form of the acid is selected from the group consisting of acyl halide, succinimidylester, acylisourea, p-$NO_2$-phenylester and methylester.

**15.** The process according to claim 11, characterised in that said base is selected from the group consisting of $K_2CO_3$, KOH, $Et_3N$ and N-methyl- morpholine.

**16.** Use of an amide of formula (I) as active principles in the preparation of pharmaceutical compositions for the therapeutic treatment of human and animal pathologies connected with the modulation of CB2 peripheral receptor, and with the degranulation and/or lysis of immunocompetent cells controlled by CB2 receptor

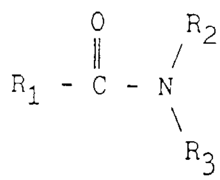 (I)

wherein $R_1$ can be:

1) a linear or branched hydrocarbon radical having from 9 to 23 carbon atoms, saturated or presenting one double bond, optionally substituted with one or more -OH groups;
2) a group of formula (II):

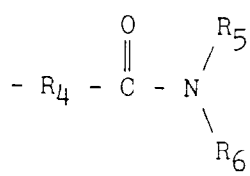 (II)

wherein $R_4$ is a linear hydrocarbon radical, saturated or containing one double bond, comprising from 7 to 22 carbon atoms, optionally substituted with one or more linear or branched alkyl groups $C_1$-$C_8$ and/or with one or more -OH groups;
3) a group of formula (IV):

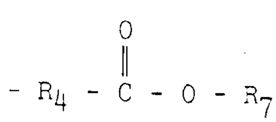 (IV)

wherein $R_4$ has the meanings described above and $R_7$ = -H or a linear or branched alkyl group, comprising

from 1 to 20 carbon atoms; the residues of formulae

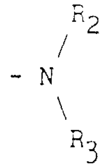 (III) and 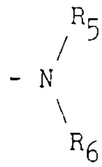 (V)

, equal or different from each other, are glycosaminic residues of formula:

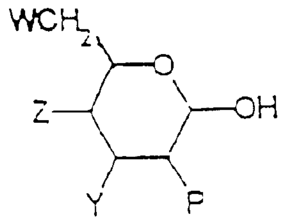

wherein one of the groups P, Y and Z is

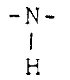 or 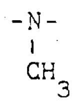 ,

and the remaining groups, including W, are -H or -OH; the residues $R_2$, $R_3$, $R_5$ and/or $R_6$ of formulae (III) and (V) being optionally substituted with at least a pharmaceutically acceptable radical capable of increasing the solubility of the amide of formula (I), said radical being selected from acyls, hemiacyls of dicarboxylic acids, dialkylaminoacyls alkyl sulphonates, alkyls O-phosphates and O-sulphates.

**17.** The use according to claim 16, characterised in that said human pathologies are pathologies connected with the immune system alteration.

**18.** The use according to claim 16, characterised in that said human pathologies are pathologies with etiologic autoimmune component, selected from the group consisting of multiple sclerosis, lateral amyothrophic sclerosis and the associated muscular spasm.

**19.** The use according to claim 16, characterised in that said human pathologies are articular acute and chronic inflammatory pathologies.

**20.** The use according to claim 19, characterised in that said chronic inflammatory pathology is rheumatoid arthritis.

**21.** The use according to claim 16, characterised in that said human pathologies are pathologies caused by biological agents selected from the group consisting of viral (HIV) and bacterial encephalitic meningitis, bacterial meningitis, meningitis by cytomegalovirus and AIDS-dementia complex.

**22.** The use according to claim 16, characterised in that said human pathologies are chronic neurodegenerative pathologies selected from the group consisting of senile dementia, Alzheimer's and Parkinson's diseases, and the associated cachectic symptomatology.

**23.** The use according to claim 16, characterised in that said human pathologies are pathologies with nociception alteration.

**24.** The use according to claim 16, characterised in that said human pathologies are pathologies associated with EAA excitotoxicity, selected from the group consisting of cerebral ictus, TIA, cerebral and spinal cord trauma, epilepsy,

migraine and chorea.

**25.** The use according to claim 16, characterised in that said human pathologies are cardiovascular pathologies associated with vascular remodelling, atherosclerosis or heart attack.

**26.** The use according to claim 16, characterised in that said human pathologies are pathologies involving pressure alterations at cardiovascular, pulmonary or ocular level.

**27.** The use according to claim 26, characterised in that said pathology is glaucoma.

**28.** The use according to claim 16, characterised in that said human pathologies are chronic airway obstructions.

**29.** The use according to claim 28, characterised in that said pathology is asthma.

**30.** The use according to claim 16, characterised in that said human pathology is nausea.

**31.** A pharmaceutical composition containing as the active principle a therapeutically active amount of amides of formula (I):

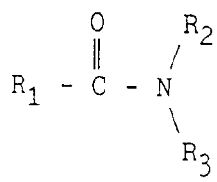

(I)

wherein $R_1$ can be:

1) a linear or branched hydrocarbon radical having from 9 to 23 carbon atoms, saturated or presenting one double bond, optionally substituted with one or more -OH groups;
2) a group of formula (II):

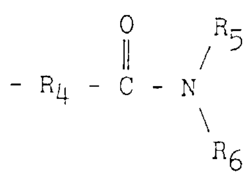

(II)

wherein $R_4$ is a linear hydrocarbon radical, saturated or containing one double bond, comprising from 7 to 22 carbon atoms, optionally substituted with one or more linear or branched alkyl groups $C_1$-$C_8$ and/or with one or more -OH groups;
3) a group of formula (IV):

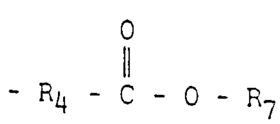

(IV)

wherein $R_4$ has the meanings described above and $R_7$ = -H or a linear or branched alkyl group, comprising from 1 to 20 carbon atoms; the residues of formulae:

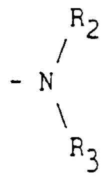 (III) and 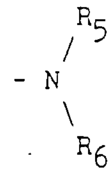 (V)

equal or different from each other, are glycosaminic residues of formula:

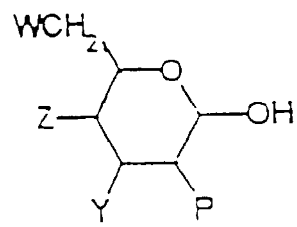

wherein one of the groups P, Y and Z is

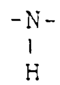 or 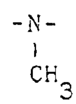 ,

and the remaining groups, including W, are -H or -OH; the residues $R_2$, $R_3$, $R_5$ and/or $R_6$ of formulae (III) and (V) being optionally substituted with at least a pharmaceutically acceptable radical capable of increasing the solubility of the amide of formula (I) said radical being selected from acyls, hemiacyls of dicarboxylic acids, dialkylaminoacyls alkyl sulphonates, alkyls, O-phosphates and O-sulphates; in combination with pharmaceutically acceptable excipients.

**32.** The pharmaceutical composition according to claim 31, wherein said active principle is administered in quantities ranging from 0.1 and 20 mg/kg/die.

**33.** The pharmaceutical composition according to claim 31, characterised in being administered by oral, parenteral, topical or transdermal route.

**34.** The pharmaceutical composition according to claim 33, characterised in that said parenteral route is endovenous, subcutaneous or intramuscular route, and said composition is in the form of injectable solution or suspension.

**35.** The pharmaceutical composition according to claim 33, characterised in that said topic route is the ophthalmic route, and said composition is in the form of eyewash, solid or semi-solid formulation in the form of insert, gel or ointment.

**36.** The pharmaceutical composition according to claim 33, characterised in being orally administered in the form of granular powder, tablets, dragees or capsules.

**37.** The pharmaceutical composition according to claim 33, characterised in being dermically or transdermically administered in the form of cream, ointment, gel or plaster, said active principle being optionally contained is slow-releasing microspheres.

**38.** The pharmaceutical composition according to claim 31, useful in the therapeutic treatment of human and animal pathologies connected with the modulation of cannabinoids peripheral receptor CB2, or with the degranulation and/or lysis of immunocompetent cells controlled by CB2 receptor.

**Patentansprüche**

**1.** Amid der Formel (I)

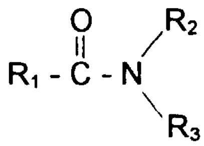

(I)

worin $R_1$ darstellen kann:

1) einen linearen oder verzweigten Kohlenwasserstoff-Rest mit 9 bis 23 Kohlenstoffatomen, der gesättigt ist oder eine Doppelbindung aufweist und gegebenenfalls durch eine oder mehrere OH-Gruppen substituiert ist;
2) eine Gruppe der Formel (II)

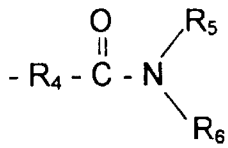

(II)

worin $R_4$ für einen linearen Kohlenwasserstoff-Rest mit 7 bis 22 Kohlenstoffatomen steht, der gesättigt ist oder eine Doppelbindung enthält und gegebenenfalls substituiert ist durch eine oder mehrere lineare oder verzweigte $C_1$-$C_8$-Alkylgruppen und/oder durch eine oder mehrere OH-Gruppen;
3) eine Gruppe der Formel (IV)

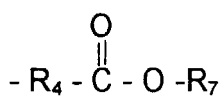

(IV)

worin $R_4$ die vorstehend angegebenen Bedeutungen hat und $R_7$ für -H oder eine lineare oder verzweigte Alkylgruppe mit 1 bis 20 Kohlenstoffatomen steht; die Reste der Formeln

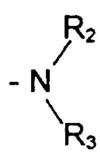

(III) und

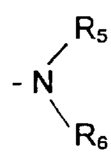

(V)

die gleich oder voneinander verschieden sind, Glycosaminreste der Formel darstellen

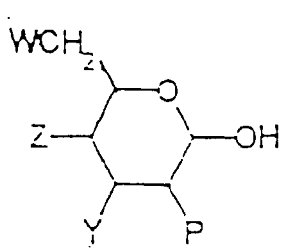

worin eine der Gruppen P, Y und Z für

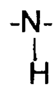 oder 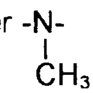

steht und die übrigen Gruppen einschließlich W -H oder -OH bedeuten; die Reste $R_2$, $R_3$, $R_5$ und/oder $R_6$ der Formeln (III) und (V) gegebenenfalls substituiert sind durch mindestens einen pharmazeutisch akzeptablen Rest, der die Löslichkeit des Amids der Formel (I) erhöhen kann, wobei der genannte Rest ausgewählt wird aus Acylen, Hemiacylen von Dicarbonsäuren, Dialkylaminoacylen, Alkylsulfonaten, Alkylen, O-Phosphaten und O-Sulfaten, mit der Maßgabe, daß das genannte Amid der Formel (I) verschieden ist von 2-Deoxy-2-[(3-hydroxy-1 -oxotetradecyl )-amino]-α-D-glucopyranose.

**2.** Amid nach Anspruch 1, dadurch gekennzeichnet, daß dann, wenn $R_1$ zur Klasse (1) gehört, dieses für einen Rest mit 11 bis 17 Kohlenstoffatomen steht.

**3.** Amid nach Anspruch 1, dadurch gekennzeichnet, daß dann, wenn $R_1$ zur Klasse (2) gehört, $R_4$ für einen Rest mit 10 bis 16 Kohlenstoffatomen steht.

**4.** Amid nach Anspruch 1, dadurch gekennzeichnet, daß das genannte Acyl Acetyl oder Benzoyl ist.

**5.** Amid nach Anspruch 1, dadurch gekennzeichnet, daß das genannte Hemiacyl einer Dicarbonsäure das Hemiacyl von Bernsteinsäure oder Glutarsäure ist.

**6.** Amid nach Anspruch 1, dadurch gekennzeichnet, daß das genannte Alkylsulfonat Methansulfonat oder p-Toluolsulfonat ist.

**7.** Amid nach Anspruch 1, dadurch gekennzeichnet, daß das genannte Alkyl Methyl oder Ethyl ist.

**8.** Amid nach Anspruch 1, dadurch gekennzeichnet, daß dann, wenn $R_1$ zur Klasse (1) gehört, dieses zusammen mit den benachbarten Carbonylen die Acylgruppe einer Monocarbonsäure bildet, die ausgewählt wird aus der Gruppe, die besteht aus Laurinsäure, Myristinsäure, Palmitinsäure, Stearinsäure, Palmitoleinsäure, Ölsäure und ω-Hydroxypalmitinsäure.

**9.** Amid nach Anspruch 1, dadurch gekennzeichnet, daß dann, wenn $R_1$ zu den Klassen (2) und (3) gehört, $R_4$ zusammen mit den beiden benachbarten Carbonylen die Acylgruppe der Traumatinsäure bildet.

**10.** Amid nach Anspruch 1, dadurch gekennzeichnet, daß das genannte Glucosamin ausgewählt wird aus der Gruppe, die besteht aus D-Glucosamin, L-Acosamine,D-Mannosamin und D-Galactosamin.

**11.** Verfahren zur Herstellung von Amiden der Formel (I)

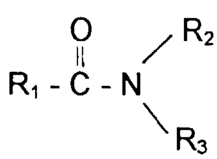 (I)

worin $R_1$ darstellen kann:

1) einen linearen oder verzweigten Kohlenwasserstoff-Rest mit 9 bis 23 Kohlenstoffatomen, der gesättigt ist oder eine Doppelbindung aufweist und gegebenenfalls durch eine oder mehrere OH-Gruppen substituiert ist;
2) eine Gruppe der Formel (II)

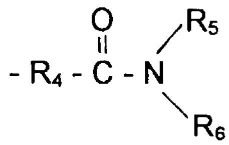

(II)

worin $R_4$ für einen linearen Kohlenwasserstoff-Rest mit 7 bis 22 Kohlenstoffatomen steht, der gesättigt ist oder eine Doppelbindung enthält und gegebenenfalls substituiert ist durch eine oder mehrere lineare oder verzweigte $C_1$-$C_8$-Alkylgruppen und/oder eine oder mehrere OH-Gruppen;

3) eine Gruppe der Formel (IV)

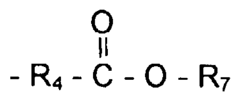

(IV)

worin $R_4$ die vorstehend angegebenen Bedeutungen hat und $R_7$ für -H oder eine lineare oder verzweigte Alkylgruppe mit 1 bis 20 Kohlenstoffatomen steht; die Reste der Formeln

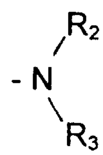

(III) und

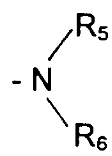

(V)

die gleich oder voneinander verschieden sind, Glycosaminreste der Formel darstellen

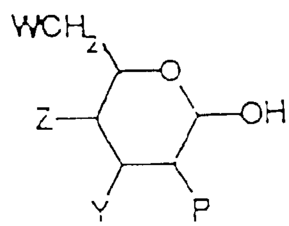

worin eine der Gruppen P, Y und 7 für

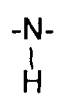

oder

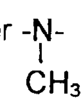

steht und die übrigen Gruppen einschließlich W für -H oder -OH stehen; die Reste $R_2$, $R_3$, $R_5$ und/oder $R_6$ der Formeln (III) und (V) gegebenenfalls durch mindestens einen pharmazeutisch akzeptablen Rest substituiert sind, der die Löslichkeit des Amids der Formel (I) erhöhen kann, wobei der genannte Rest ausgewählt wird aus Acylen, Hemiacylen von Dicarbonsäuren, Dialkylaminoacylen, Alkylsulfonaten, Alkylen, O-Phosphaten und O-Sulfaten; mit der Maßgabe, daß das genannte Amid der Formel (I) von 2-Deoxy-2-[(3-hydroxy-1 -oxotetradecyl)-amino]α-D-glucopyranose verschieden ist, wobei das Verfahren umfaßt die Umsetzung einer aktivierten Form der Säure $R_1$-COOH mit der Formel $R_1$-CO-X mit einem Amin der Formel

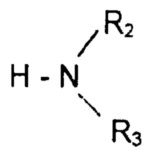

in wäßrigen oder organischen Lösungsmitteln in Gegenwart einer Base unter Rühren bei einer Temperatur in dem Bereich von -20 bis 60°C für eine Reaktionszeit in dem Bereich von 10 min bis 24 h;
gegebenenfalls das Ansäuern des Reaktionsmediums; und
das Isolieren und Reinigen unter Anwendung konventioneller Methoden.

**12.** Verfahren nach Anspruch 11, dadurch gekennzeichnet, daß die genannte Temperatur zwischen -10 und 20°C liegt.

**13.** Verfahren nach Anspruch 11, dadurch gekennzeichnet, daß die genannte Reaktionszeit zwischen 1 h und 3 h liegt.

**14.** Verfahren nach Anspruch 11, dadurch gekennzeichnet, daß die genannte aktivierte Form der Säure ausgewählt wird aus der Gruppe, die besteht aus dem Acylhalogenid, Succinimidylester, Acylisoharnstoff, p-$NO_2$-Phenylester und Methylester.

**15.** Verfahren nach Anspruch 11, dadurch gekennzeichnet, daß die genannte Base ausgewählt wird aus der Gruppe, die besteht aus $K_2CO_3$, KOH, $Et_3N$ und N-Methyl-morpholin.

**16.** Verwendung eines Amids der Formel (I) als Wirkstoff (aktives Prinzip) bei der Herstellung von pharmazeutischen Zusammensetzungen zur therapeutischen Behandlung von Human- und Tier-Pathologien, die mit der Modulation des peripheren CB2-Rezeptors und mit der Degranulation und/oder Lysis von immunkompetenten Zellen, die durch den CB2-Rezeptor kontrolliert wird, im Zusammenhang stehen

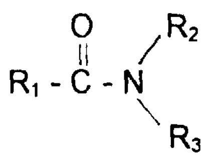 (I)

worin $R_1$ darstellen kann:

1) einen linearen oder verzweigten Kohlenwasserstoff-Rest mit 9 bis 23 Kohlenstoffatomen, der gesättigt ist oder eine Doppelbindung aufweist und gegebenenfalls durch eine oder mehrere OH-Gruppen substituiert ist;
2) eine Gruppe der Formel (II)

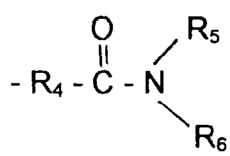 (II)

worin $R_4$ für einen linearen Kohlenwasserstoff-Rest mit 7 bis 22 Kohlenstoffatomen steht, der gesättigt ist oder eine Doppelbindung enthält und gegebenenfalls substituiert ist durch eine oder mehrere lineare oder verzweigte $C_1$-$C_8$-Alkylgruppen und/oder eine oder mehrere OH-Gruppen;
3) eine Gruppe der Formel (IV)

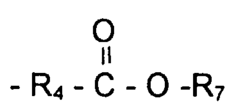 (IV)

worin $R_4$ die vorstehend angegebenen Bedeutungen hat und $R_7$ für -H oder eine lineare oder verzweigte Alkylgruppe mit 1 bis 20 Kohlenstoffatomen steht; die Reste der Formeln

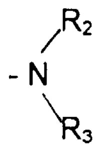 (III) und 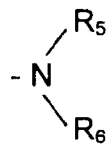 (V)

die gleich oder voneinander verschieden sind, Glycosaminreste der Formel darstellen

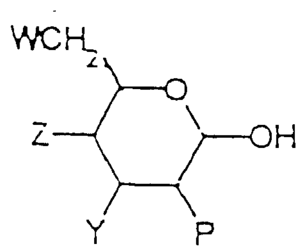

worin eine der Gruppen P, Y und Z für

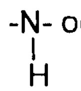 oder 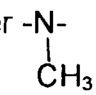

steht und die übrigen Gruppen einschließlich W für -H oder -OH stehen;
die Reste $R_2$, $R_3$, $R_5$ und/oder $R_6$ der Formeln (III) und (V) gegebenenfalls substituiert sind durch mindestens einen pharmazeutisch akzeptablen Rest, der die Löslichkeit des Amids der Formel (I) erhöhen kann, wobei der genannte Rest ausgewählt wird aus Acylen, Hemiacylen von Dicarbonsäuren, Dialkylaminoacylen, Alkylsulfonaten, Alkylen, O-Phosphaten und O-Sulfaten.

**17.** Verwendung nach Anspruch 16, dadurch gekennzeichnet, daß es sich bei den genannten Human-Pathologien um Pathologien handelt, die im Zusammenhang stehen mit einer Immunsystem-Alteration.

**18.** Verwendung nach Anspruch 16, dadurch gekennzeichnet, daß die genannten Human-Pathologien Pathologien mit einer ätiologischen AutoimmunKomponente sind, die ausgewählt werden aus der Gruppe, die besteht aus Multipler Sklerose, amyotrophe Lateral-Sklerose und damit verbundenen Muskelspasmen.

**19.** Verwendung nach Anspruch 16, dadurch gekennzeichnet, daß es sich bei den Human-Pathologien um akute und chronische GelenkentzündungsPathologien handelt.

**20.** Verwendung nach Anspruch 19, dadurch gekennzeichnet, daß es sich bei der genannten chronischen Entzündungs-Pathologie um Rheumatoide Arthritis handelt.

**21.** Verwendung nach Anspruch 16, dadurch gekennzeichnet, daß es sich bei den genannten Human-Pathologien um Pathologien handelt, die durch biologische Agentien hervorgerufen werden, ausgewählt aus der Gruppe, die besteht aus viraler (HIV) und bakterieller encephalitischer Meningitis, bakterieller Meningitis, Meningitis, die durch Cytomegalovirus hervorgerufen wird, und dem AIDS-Dementia-Komplex.

**22.** Verwendung nach Anspruch 16, dadurch gekennzeichnet, daß es sich bei den genannten Human-Pathologien um chronische neurodegenerative Pathologien handelt, die ausgewählt werden aus der Gruppe, die besteht aus seniler Dementia, Alzheimer Krankheit und Parkinson Krankheit und der damit verbundenen kachektischen Symptomatologie.

**23.** Verwendung nach Anspruch 16, dadurch gekennzeichnet, daß es sich bei den genannten Human-Pathologien um

Pathologien mit einer Nozizeptions-Alteration handelt.

**24.** Verwendung nach Anspruch 16, dadurch gekennzeichnet, daß es sich bei den genannten Human-Pathologien um Pathologien handelt, die mit einer EAA-Excitotoxizität im Zusammenhang stehen, die ausgewählt werden aus der Gruppe, die besteht aus dem cerebralen Ictus, TIA, cerebralen und Rückenmarks-Traumata, Epilepsie, Migräne und Chorea.

**25.** Verwendung nach Anspruch 16, dadurch gekennzeichnet, daß es sich bei den genannten Human-Pathologien um cardiovasculäre Pathologien handelt, die mit einer Gefäßremodellierung, einer Arteriosklerose oder einer Herzattacke im Zusammenhang stehen.

**26.** Verwendung nach Anspruch 16, dadurch gekennzeichnet, daß es sich bei den genannten Human-Pathologien um Pathologien handelt, die Druckalterationen im cardiovasculären, Lungen- oder Augen-Bereich mit sich bringen.

**27.** Verwendung nach Anspruch 26, dadurch gekennzeichnet, daß es sich bei der genannten Pathologie um das Glaucom handelt.

**28.** Verwendung nach Anspruch 16, dadurch gekennzeichnet, daß es sich bei den genannten Human-Pathologien um eine chronische Verlegungen der Atemwege handelt.

**29.** Verwendung nach Anspruch 28, dadurch gekennzeichnet, daß es sich bei der genannten Pathologie um Asthma handelt.

**30.** Verwendung nach Anspruch 16, dadurch gekennzeichnet, daß es sich bei der genannten Human-Pathologie um Nausea handelt.

**31.** Pharmazeutische Zusammensetzung, die als Wirkstoff (aktives Prinzip) eine therapeutisch aktive Menge der Amide der Formel (I):

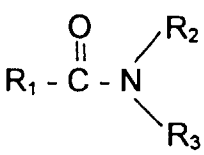

(I)

worin $R_1$ darstellen können:

1) einen linearen oder verzweigten Kohlenwasserstoff-Rest mit 9 bis 23 Kohlenstoffatomen, der gesättigt ist oder eine Doppelbindung aufweist und gegebenenfalls durch eine oder mehrere OH-Gruppen substituiert ist;
2) eine Gruppe der Formel (II)

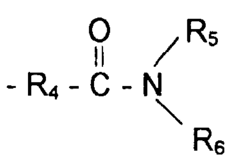

(II)

worin $R_4$ für einen linearen Kohlenwasserstoff-Rest mit 7 bis 22 Kohlenstoffatomen steht, der gesättigt ist oder eine Doppelbindung enthält und gegebenenfalls substituiert ist durch eine oder mehrere lineare oder verzweigte $C_1$-$C_8$-Alkylgruppen und/oder eine oder mehrere OH-Gruppen;
3) eine Gruppe der Formel (IV)

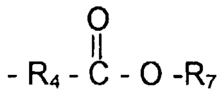

(IV)

worin $R_4$ die vorstehend angegebenen Bedeutungen hat und $R_7$ für -H oder eine lineare oder verzweigte Alkylgruppe mit 1 bis 20 Kohlenstoffatomen steht; die Reste der Formeln

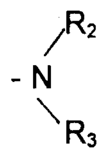 (III) und 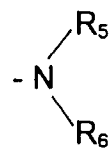 (V)

die gleich oder voneinander verschieden sind, Glycosaminreste der Formel darstellen

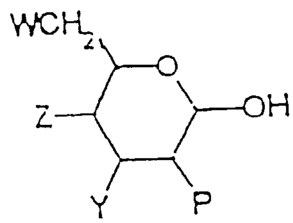

worin eine der Gruppen P, Y und Z für

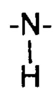 oder 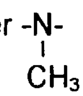

steht und die übrigen Gruppen einschließlich W für -H oder -OH stehen;
die Reste $R_2$, $R_3$, $R_5$ und/oder $R_6$ der Formeln (III) und (V) gegebenenfalls substituiert sind durch mindestens einen pharmazeutisch akzeptablen Rest, der die Löslichkeit des Amids der Formel (I) erhöhen kann, wobei der genannte Rest ausgewählt wird aus Acylen, Hemiacylen von Dicarbonsäuren, Dialkylaminoacylen, Alkylsulfonaten, Alkylen, O-Phosphaten und O-Sulfaten, in Kombination mit pharmazeutisch akzeptablen Exzipienten enthält.

**32.** Pharmazeutische Zusammensetzung nach Anspruch 31, wobei der genannte Wirkstoff (aktive Prinzip) in Mengen in dem Bereich von 0,1 bis 20 mg/kg/Tag verabreicht wird.

**33.** Pharmazeutische Zusammensetzung nach Anspruch 31, dadurch gekennzeichnet, daß sie auf oralem, parenteralem, topischem oder transdermalem Wege verabreicht wird.

**34.** Pharmazeutische Zusammensetzung nach Anspruch 33, dadurch gekennzeichnet, daß es sich bei dem genannten parenteralen Weg um den endovenösen, subkutanen oder intramuskulären Weg handelt, und daß die genannte Zusammensetzung in Form einer injizierbaren Lösung oder Suspension vorliegt.

**35.** Pharmazeutische Zusammensetzung nach Anspruch 33, dadurch gekennzeichnet, daß es sich bei dem genannten topischen Weg um den ophthalmischen Weg handelt und die genannte Zusammensetzung in Form einer Augenspülung, einer festen oder halbfesten Formulierung in Form eines Inserts, Gels oder einer Salbe vorliegt.

**36.** Pharmazeutische Zusammensetzung nach Anspruch 33, dadurch gekennzeichnet, daß sie in Form eines granulären Pulvers, in Form von Tabletten, Dragees oder Kapseln oral verabreicht wird.

**37.** Pharmazeutische Zusammensetzung nach Anspruch 33, dadurch gekennzeichnet, daß sie in Form einer Creme, einer Salbe, eines Gels oder eines Pflasters dermal oder transdermal verabreicht wird, wobei der genannte Wirkstoff (aktive Prinzip) gegebenenfalls in Mikrokugeln mit langsamer Freigabe enthalten ist.

**38.** Pharmazeutische Zusammensetzung nach Anspruch 31, die verwendbar ist zur therapeutischen Behandlung von Human- und Tierpathologien, die mit der Modulation des peripheren Cannabinoid-Rezeptors CB2 oder mit der Degranulation und/oder Lysis von durch den CB2-Rezeptor kontrollierten immunkompetenten Zellen im Zusammenhang stehen.

## Revendications

**1.** Amide de formule (I) :

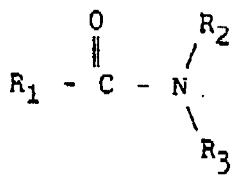

(I)

dans laquelle $R_1$ peut être :

1) un radical hydrocarboné à chaîne droite ou ramifiée ayant de 9 à 23 atomes de carbone, saturé ou présentant une double liaison, éventuellement substitué par un ou plusieurs groupes -OH ;
2) un groupe de formule (II) :

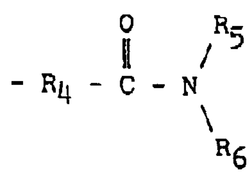

(II)

dans laquelle $R_4$ est un radical hydrocarboné linéaire, saturé ou contenant une double liaison, comprenant de 7 à 22 atomes de carbone, éventuellement substitué par un ou plusieurs groupes alkyle en $C_1$-$C_8$ à chaîne droite ou ramifiée et/ou par un ou plusieurs groupes -OH ;
3) un groupe de formule (IV):

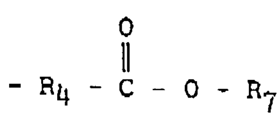

(IV)

dans laquelle $R_4$ a les significations données ci-dessus, et $R_7$ est -H ou un groupe alkyle à chaîne droite ou ramifiée, comprenant de 1 à 20 atomes de carbone ;
les résidus de formules :

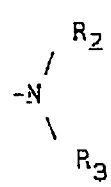

(III) et

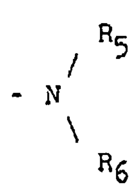

(V)

qui sont identiques l'un à l'autre ou différents l'un de l'autre, sont des résidus glycosaminiques de formule

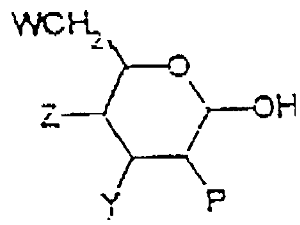

où l'un des groupes P, Y et Z est

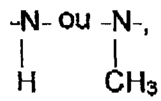

et les groupes restants, y compris W, représentent -H ou -OH ;
les résidus $R_2$, $R_3$, $R_5$ et/ou $R_6$ des formules (III) et (V) étant éventuellement substitués par au moins un radical acceptable d'un point de vue pharmaceutique, capable d'augmenter la solubilité de l'amide de formule (I), ledit radical étant choisi parmi les acyles, les hémiacyles d'acides dicarboxyliques, les dialkylaminoacyles, les alkylsulfonates, les alkyles, les O-phosphates et les O=sulfates ; à la condition que ledit amide de formule (I) soit différent du 2-désoxy-2-[(3-hydroxy-1 -oxotétradécyl)-amino]-α-D-glucopyrannose.

**2.** Amide selon la revendication 1, caractérisé en ce que, quand $R_1$ appartient à la classe (1), il s'agit d'un radical ayant de 11 à 17 atomes de carbone.

**3.** Amide selon la revendication 1, caractérisé en ce que, quand $R_1$ appartient à la classe (2), $R_4$ est un radical contenant de 10 à 16 atomes de carbone.

**4.** Amide selon la revendication 1, caractérisé en ce que ledit acyle est l'acétyle ou le benzoyle.

**5.** Amide selon la revendication 1, caractérisé en ce que ledit hémiacyle d'un acide dicarboxylique est l'hémiacyle de l'acide succinique ou de l'acide glutarique.

**6.** Amide selon la revendication 1, caractérisé en ce que ledit alkylsulfonate est un méthanesulfonate ou un p-toluènesulfonate.

**7.** Amide selon la revendication 1, caractérisé en ce que ledit alkyle est le méthyle ou l'éthyle.

**8.** Amide selon la revendication 1, caractérisé en ce que, quand $R_1$ appartient à la classe (1), il forme avec les groupes carbonyle voisins le groupe acyle d'un acide monocarboxylique choisi dans le groupe comprenant l'acide laurique, l'acide myristique, l'acide palmitique, l'acide stéarique, l'acide palmitoléique, l'acide oléique et l'acide ω-hydroxypalmitique .

**9.** Amide selon la revendication 1, caractérisé en ce que, quand $R_1$ appartient aux classes (2) et (3), $R_4$ avec les deux groupes carbonyle voisins le groupe acyle de l'acide traumatique.

**10.** Amide selon la revendication 1, caractérisé en ce que ladite glycosamine est choisie dans l'ensemble comprenant la D-glucosamine, la L-acosamine, la D-mannosamine et la D-galactosamine.

**11.** Procédé de préparation d'amides de formule (I):

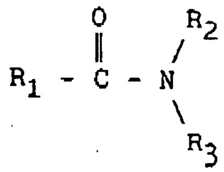 (I)

dans laquelle $R_1$ peut être :

1) un radical hydrocarboné à chaîne droite ou ramifiée ayant de 9 à 23 atomes de carbone, saturé ou présentant une double liaison, éventuellement substitué par un ou plusieurs groupes -OH ;
2) un groupe de formule (II) :

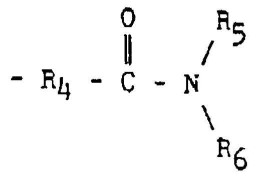 (II)

dans laquelle $R_4$ est un radical hydrocarboné linéaire, saturé ou contenant une double liaison, comprenant de 7 à 22 atomes de carbone, éventuellement substitué par un ou plusieurs groupes alkyle en $C_1$-$C_8$ à chaîne droite ou ramifiée et/ou par un ou plusieurs groupes -OH ;
3) un groupe de formule (IV):

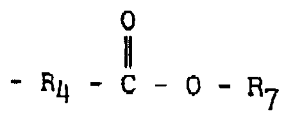 (IV)

dans laquelle $R_4$ a les significations données ci-dessus, et $R_7$ est -H ou un groupe alkyle à chaîne droite ou ramifiée, comprenant de 1 à 20 atomes de carbone ;
les résidus de formules :

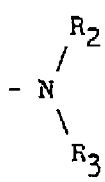 (III) et 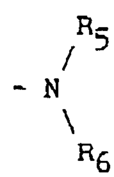 (V)

qui sont identiques l'un à l'autre ou différents l'un de l'autre, sont des résidus glycosaminiques de formule

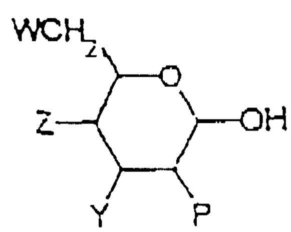

où l'un des groupes P, Y et Z est

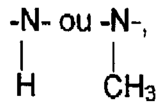

et les groupes restants, y compris W, représentent -H ou -OH ;
les résidus $R_2$, $R_3$, $R_5$ et/ou $R_6$ des formules (III) et (V) étant éventuellement substitués par au moins un radical acceptable d'un point de vue pharmaceutique, capable d'augmenter la solubilité de l'amide de formule (I), ledit radical étant choisi parmi les acyles, les hémiacyles d'acides dicarboxyliques, les dialkylaminoacyles, les alkylsulfonates, les alkyles, les O-phosphates et les O=sulfates ; à la condition que ledit amide de formule (I) soit différent du 2-désoxy-2-[(3-hydroxy-1-oxotétradécyl)-amino]-α-D-glucopyrannose;
qui comprend la réaction d'une forme activée de l'acide $R_1$-COOH, de formule $R_1$-CO-X, avec une amine de formule

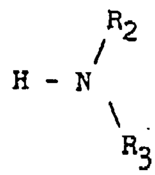

dans des solvants aqueux ou organiques, en présence d'une base, sous agitation à une température comprise entre -20 et +60°C, pendant un laps de temps compris entre 10 minutes et 24 heures ; l'éventuelle acidification du milieu réactionnel ; l'isolement et la purification par des techniques classiques.

**12.** Procédé selon la revendication 11, caractérisé en ce que ladite température est comprise entre -10 et 20°C.

**13.** Procédé selon la revendication 11, caractérisé en ce que la durée de la réaction est comprise entre 1 heure et 3 heures.

**14.** Procédé selon la revendication 11, caractérisé en ce que ladite forme activée de l'acide est choisie dans l'ensemble comprenant les halogénures d'acyle, les esters succinimidyliques, les acyl-isourées, les esters p-$NO_2$-phényliques et les esters méthyliques.

**15.** Procédé selon la revendication 11, caractérisé en ce que ladite base est choisie dans l'ensemble comprenant $K_2CO_3$, KOH, $Et_3N$ et la N-méthylmorpholine.

**16.** Utilisation d'un amide de formule (I) en tant que principe actif lors de la préparation de compositions pharmaceutiques destinées au traitement thérapeutique de pathologies humaines et animales en liaison avec une modulation du récepteur périphérique CB2, et avec la dégranulation et/ou la lyse de cellules immunocompétentes, commandée par le récepteur CB2

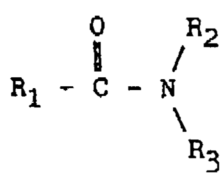

(I)

dans laquelle $R_1$ peut être :

1) un radical hydrocarboné à chaîne droite ou ramifiée ayant de 9 à 23 atomes de carbone, saturé ou présentant une double liaison, éventuellement substitué par un ou plusieurs groupes -OH ;
2) un groupe de formule (II) :

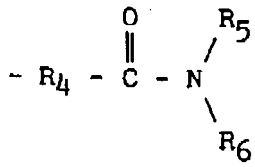 (II)

dans laquelle $R_4$ est un radical hydrocarboné linéaire, saturé ou contenant une double liaison, comprenant de 7 à 22 atomes de carbone, éventuellement substitué par un ou plusieurs groupes alkyle en $C_1$-$C_8$ à chaîne droite ou ramifiée et/ou par un ou plusieurs groupes -OH ;

3) un groupe de formule (IV):

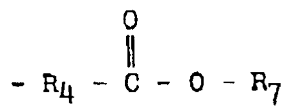 (IV)

dans laquelle $R_4$ a les significations données ci-dessus, et $R_7$ est -H ou un groupe alkyle à chaîne droite ou ramifiée, comprenant de 1 à 20 atomes de carbone ;

les résidus de formules :

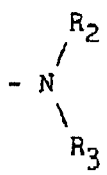 (III) et 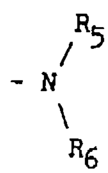 (V)

qui sont identiques l'un à l'autre ou différents l'un de l'autre, sont des résidus glycosaminiques de formule

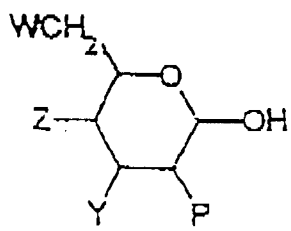

où l'un des groupes P, Y et Z est

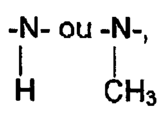

ou

et les groupes restants, y compris W, représentent -H ou -OH ;

les résidus $R_2$, $R_3$, $R_5$ et/ou $R_6$ des formules (III) et (V) étant éventuellement substitués par au moins un radical acceptable d'un point de vue pharmaceutique, capable d'augmenter la solubilité de l'amide de formule (I), ledit radical étant choisi parmi les acyles, les hémiacyles d'acides dicarboxyliques, les dialkylaminoacyles, les alkylsulfonates, les alkyles, les O-phosphates et les O=sulfates.

**17.** Utilisation selon la revendication 16, caractérisée en ce que lesdites pathologies humaines sont des pathologies en liaison avec un altération du système immun.

**18.** Utilisation selon la revendication 16, caractérisée en ce que lesdites pathologies humaines sont des pathologies comportant une composante auto-immune étiologique, choisies dans l'ensemble comprenant la sclérose en plaque, la sclérose amyotrophique latérale et les spasmes musculaires associés.

**19.** Utilisation selon la revendication 16, caractérisée en ce que lesdites pathologies humaines sont des pathologies inflammatoires articulaires aiguës et chroniques.

**20.** Utilisation selon la revendication 19, caractérisée en ce que ladite pathologie inflammatoire chronique est la polyarthrite rhumatoïde.

**21.** Utilisation selon la revendication 16, caractérisée en ce que lesdites pathologies humaines sont des pathologies provoquées par des agents biologiques choisis dans l'ensemble comprenant la méningite encéphalitique d'origine virale (VIH) ou bactérienne, la méningite bactérienne, la méningite à cytomégalovirus et le complexe SIDA-démence.

**22.** Utilisation selon la revendication 16, caractérisée en ce que lesdites pathologies humaines sont des pathologies neurodégénératives chroniques choisies dans l'ensemble comprenant la démence sénile, la maladie d'Alzheimer et la maladie de Parkinson, et la symptomatologie cachectique associée.

**23.** Utilisation selon la revendication 16, caractérisée en ce que lesdites pathologies humaines sont des pathologies présentant une altération de la nociception.

**24.** Utilisation selon la revendication 16, caractérisée en ce que lesdites pathologies humaines sont des pathologies associées à l'excitotoxicité des EAA, choisies dans l'ensemble comprenant l'ictus cérébral, l'accident cérébrovasculaire transitoire de nature ischémique, les traumatismes du cerveau et de la moelle épinière, l'épilepsie, la migraine et la chorée.

**25.** Utilisation selon la revendication 16, caractérisée en ce que lesdites pathologies humaines sont des pathologies cardiovasculaires associées à un remodelage vasculaire, à une athérosclérose ou à une attaque cardiaque.

**26.** Utilisation selon la revendication 16, caractérisée en ce que lesdites pathologies humaines sont des pathologies mettant en jeu des altérations de la pression au niveau cardiovasculaire, pulmonaire ou oculaire.

**27.** Utilisation selon la revendication 26, caractérisée en ce que ladite pathologie est le glaucome.

**28.** Utilisation selon la revendication 16, caractérisée en ce que lesdites pathologies humaines sont des obstructions chroniques des voies respiratoires.

**29.** Utilisation selon la revendication 28, caractérisée en ce que ladite pathologie est l'asthme.

**30.** Utilisation selon la revendication 16, caractérisée en ce que ladite pathologie humaine est la nausée.

**31.** Composition pharmaceutique contenant en tant que principe actif une quantité à effet thérapeutique d'amides de formule (I) :

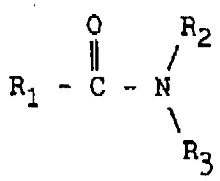

(I)

dans laquelle $R_1$ peut être:

1) un radical hydrocarboné à chaîne droite ou ramifiée ayant de 9 à 23 atomes de carbone, saturé ou présentant une double liaison, éventuellement substitué par un ou plusieurs groupes -OH ;
2) un groupe de formule (II) :

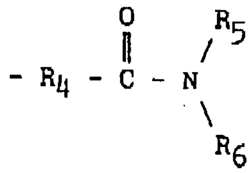

(II)

dans laquelle $R_4$ est un radical hydrocarboné linéaire, saturé ou contenant une double liaison, comprenant de 7 à 22 atomes de carbone, éventuellement substitué par un ou plusieurs groupes alkyle en $C_1$-$C_8$ à chaîne droite ou ramifiée et/ou par un ou plusieurs groupes -OH ;
3) un groupe de formule (IV) :

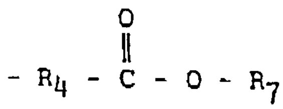

(IV)

dans laquelle $R_4$ a les significations données ci-dessus, et $R_7$ est -H ou un groupe alkyle à chaîne droite ou ramifiée, comprenant de 1 à 20 atomes de carbone ; les résidus de formules :

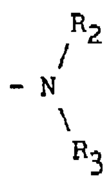

(III) et

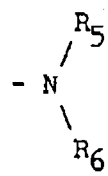

(V)

qui sont identiques l'un à l'autre ou différents l'un de l'autre, sont des résidus glycosaminiques de formule

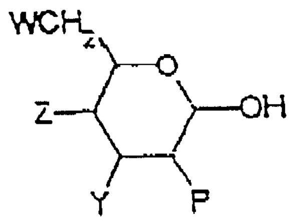

où l'un des groupes P, Y et Z est

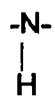

ou

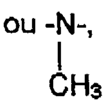

et les groupes restants, y compris W, représentent -H ou -OH ;
les résidus $R_2$, $R_3$, $R_5$ et/ou $R_6$ des formules (III) et (V) étant éventuellement substitués par au moins un radical acceptable d'un point de vue pharmaceutique, capable d'augmenter la solubilité de l'amide de formule (I), ledit radical étant choisi parmi les acyles, les hémiacyles d'acides dicarboxyliques, les dialkylaminoacyles, les alkylsulfonates, les alkyles, les O-phosphates et les O=sulfates ; en combinaison avec des excipients acceptables d'un point de vue pharmaceutique.

**32.** Composition pharmaceutique selon la revendication 31, dans laquelle ledit principe actif est administré en des quantités comprises entre 0,1 et 20 mg/kg/jour.

**33.** Composition pharmaceutique selon la revendication 31, caractérisée en ce qu'elle est administrée par voie orale,

parentérale, topique ou transdermique.

**34.** Composition pharmaceutique selon la revendication 33, caractérisée en ce que ladite voie parentérale est la voie intraveineuse, sous-cutanée ou intramusculaire, et ladite composition se présente sous forme d'une solution ou d'une suspension injectable.

**35.** Composition pharmaceutique selon la revendication 33, caractérisée en ce que ladite voie topique est la voie ophtalmique, et ladite composition se présente sous forme d'un collyre, d'une formulation solide ou semi-solide sous forme d'un insert, d'un gel ou d'une pommade.

**36.** Composition pharmaceutique selon la revendication 33, caractérisée en ce qu'elle est administrée par voie orale sous forme de granulés, d'une poudre, de comprimés, de dragées ou de gélules.

**37.** Composition pharmaceutique selon la revendication 33, caractérisée en ce qu'elle est administrée par voie dermique ou transdermique sous forme d'une crème, d'une pommade, d'un gel ou d'un emplâtre, ledit principe actif étant éventuellement contenu dans des microsphères à libération retard.

**38.** Composition pharmaceutique selon la revendication 31, pouvant être utilisée dans le traitement thérapeutique de pathologies humaines et animales en liaison avec la modulation du récepteur périphérique CB2 des cannabinoïdes, ou avec la dégranulation et/ou la lyse de cellules immunocompétentes, commandée par le récepteur CB2.

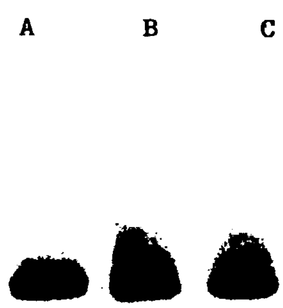


FIG. 1

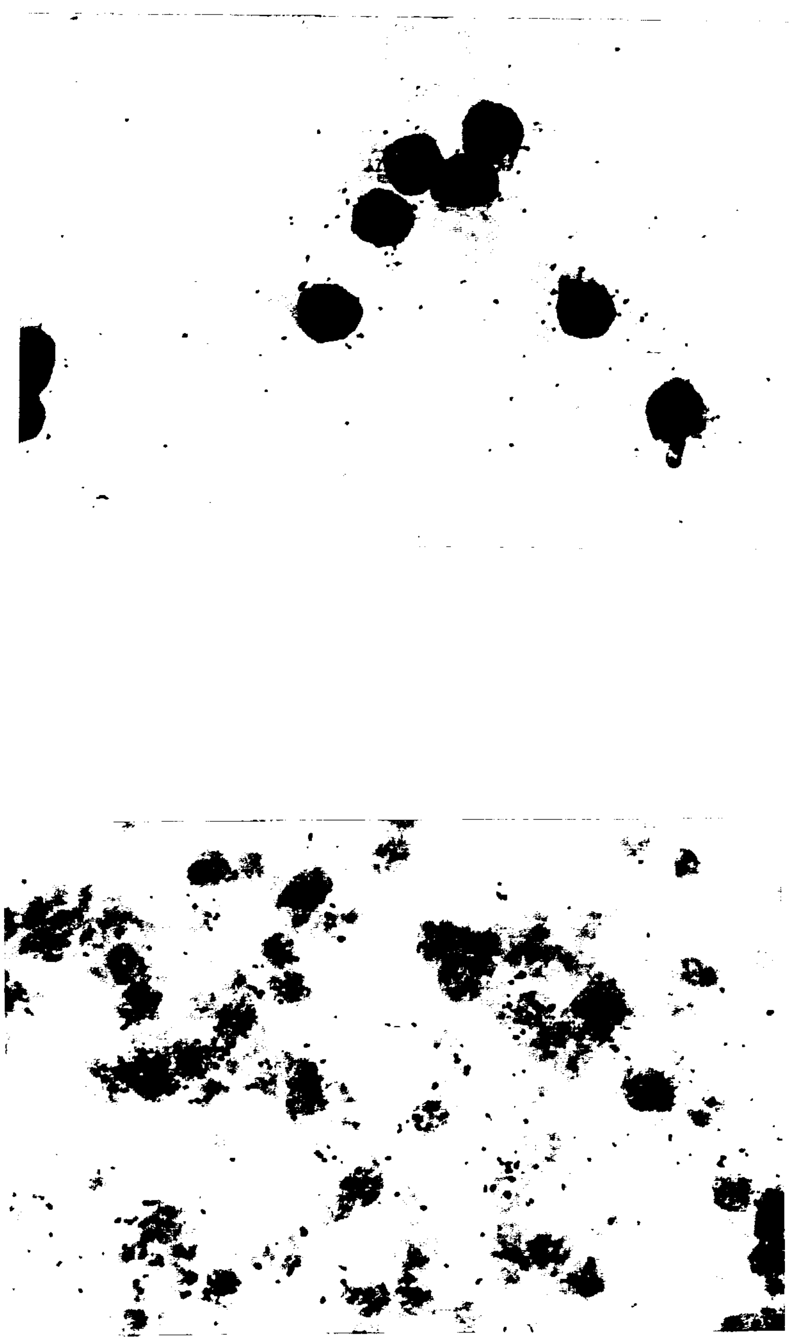

A

B

FIG. 2